# FASCICULE DE BREVET EUROPEEN

(11) **EP 1 877 500 B1**
(45) Date de publication et mention de la délivrance du brevet: **22.10.2008**
(21) Numéro de dépôt: 06755469.1
(22) Date de dépôt: 02.05.2006
(51) Int. Cl.: C09C 3/10, C08K 3/22, B01F 3/20, B01F 17/00

(54) **ORGANOSOL STABILISE PAR DES POLYMERES SEQUENCES AMPHIPHILES**
MITTELS AMPHIPHILEN BLOCKCOPLYMEREN STABILISIERTES ORGANOSOL
ORGANOSOL STABILIZED BY AMPHIPHILIC BLOCK POLYMERS

(30) Priorité: 03.05.2005 FR 0504511
(43) Date de publication de la demande: 16.01.2008
(73) Titulaire: RHODIA CHIMIE, 93300 Aubervilliers Cedex (FR)
(72) Inventeur: DESTARAC, Mathias, F-75005 Paris (FR); PAVAGEAU, Bertrand, F-33140 Villenave D'Ornon (FR); TOLLA, Bruno, F-75005 Paris (FR)
(74) Mandataire: Colombet, Alain André
(86) Numéro de dépôt international: PCT/FR2006/000984
(87) Numéro de publication internationale: WO 2006/117476

(56) Documents cités:
- WO-A-03/090916
- FR-A- 2 789 601
- FR-A- 2 845 930
- US-A- 5 718 907
- US-A- 6 153 705
- US-B1- 6 777 513

## Description

La présente invention a trait à des dispersions stabilisées de particules minérales, dans lesquelles le milieu dispersant est un milieu organique, de préférence un milieu organique hydrophobe. Ces dispersions sont généralement désignées par le terme d'organosols (sols organiques).

Un problème majeur rencontré avec de tels organosols est celui de l'incompatibilité entre les particules, de nature inorganique, et le milieu dispersant, organique et le plus souvent hydrophobe. En effet, à de très rares exceptions près, le milieu dispersant présente une très faible affinité pour la surface des particules inorganiques, généralement hydrophile.

Ce problème d'incompatibilité s'avère tout particulièrement prononcé lorsque les particules présentent une charge électrique de surface, comme c'est notamment le cas pour les particules à base d'oxyde minéral, dont la surface est généralement porteuse d'espèces cationiques ou anioniques. Lorsque de telles charges de surface sont présentes, les particules tendent à s'agréger, ce qui conduit en règle générale à une déstabilisation de l'organosol, et ce tout particulièrement lorsque la taille des particules est réduite, par exemple lorsque les particules de l'organosol ont des dimensions moyennes inférieures à 1 µm, par exemple comprises entre 1 nm et 500 nm, les particules développant alors une surface d'échange importante avec le milieu organique.

Pour éviter les inconvénients précités, on a proposé différents types de modification de la surface des particules envisagés pour les organosols, de façon à les compatibiliser avec le milieu organique dispersant.

Une première solution proposée dans ce cadre consiste à greffer des chaînes organiques à la surface des particules de façon à modifier leur surface et à éviter l'agrégation interparticulaire. A cet effet, il a par exemple été proposé, notamment dans la demande FR 2 721 615, de modifier la surface des particules par des agents de type silane, ce qui permet de greffer de façon covalente des chaînes organiques à la surface des particules que l'on souhaite stabiliser. Cette solution s'avère généralement efficace, mais elle se révèle le plus souvent complexe à mettre en oeuvre, et, compte tenu des silanes les plus usuellement disponibles, elle est généralement limitée à la préparation d'organosols où le milieu dispersant est apolaire. En outre, la mise en oeuvre de silanes conduit à une génération d'alcools (éthanol ou méthanol par exemple) à titre de sous-produits, qui peuvent s'avérer gênants dans certaines applications.

Pour éviter cette génération d'alcool, une solution alternative à l'emploi des silanes, décrite notamment par Meriguet et coll. dans le Journal of Colloid and Interface Science, 267, pp. 78-85 (2003), consiste à utiliser des agents de type tensioactifs dans la dispersion.

Dans ce cadre, on connaît en particulier de FR 2716 388 l'utilisation d'acides carboxyliques amphiphiles, tels que l'acide laurique ou l'acide isostéarique, pour préparer des organosols à base de particules de CeO₂. Pour la stabilisation d'organosols, on a également proposé des agents tensioactifs cationiques du type du bromure de diméthyl-didodécyl-ammonium, et, plus récemment, des tensioactifs anioniques, comme par exemple le dodécylbenzenesulfonate de sodium, utilisé par Ramakrishna et coll. pour la stabilisation d'organosols à base de particules de TiO2 (Langmuir, vol. 19, pp. 505-508 (2003)).

Les sols obtenus en mettant en oeuvre les agents tensioactifs précédents sont, en général, relativement bien stables. Toutefois, cette stabilité présente des limites, et ce notamment lorsque l'organosol est stocké ou utilisé sur de longues périodes, par exemple lorsqu'il est employé à des température élevées, par exemple de l'ordre de 70 à 80°C, et tout particulièrement lorsqu'il est soumis à des fluctuations importantes de température. Dans ces conditions, on observe à plus ou moins long terme une déstabilisation de l'organosol, qui conduit à une agrégation et à une floculation des particules (cette déstabilisation peut par exemple être mise en évidence en soumettant l'organosol à des cycles de montée et descente en température entre -20°C et 90°C). Pour beaucoup d'applications, la stabilité obtenue avec un tensioactif de type acide isostéarique s'avère satisfaisante, mais on cherche néanmoins à améliorer encore cette stabilité, en particulier pour certaines applications spécifiques, où une stabilité à plus long terme est requise.

De plus, la stabilité des organosols stabilisés par des agents tensioactifs est souvent affectée par l'introduction de certains additifs de formulation qui ont tendance à fragiliser le sol, ce qui peut interdire l'utilisation de ces additifs et limite par conséquent les domaines d'application de l'organosol.

En outre, l'utilisation des tensioactifs précités ne permet en général pas la stabilisation d'organosols à base de milieux dispersants polaires.

Un but de l'invention est de fournir des organosols plus stables que les organosols précités à base de tensioactifs, à savoir des organosols qui soient stables sur de longues périodes de stockage, et capables en particulier de résister à des traitements thermiques à des températures de l'ordre de 70 à 80°C comme à des variations de températures importantes, sans que la structure de la dispersion ne soit sensiblement affectée. L'invention vise à atteindre ce but sans avoir à mettre en oeuvre un greffage covalent de chaînes organiques sur la surface des particules.

Dans ce cadre, l'invention vise plus spécifiquement à fournir des organosols stabilisés qui puissent contenir un milieu dispersant aussi bien polaire qu'apolaire, et qui permettent de préférence d'éviter l'ensemble des problèmes précités rencontrés avec les organosols actuellement connus.

A cet effet, selon un premier aspect, la présente invention a pour objet une dispersion stabilisée de particules minérales au sein d'un milieu dispersant organique (c'est-à-dire un organosol de ces particules minérales), qui contient, à titre d'agents de stabilisation de la dispersion, des polymères séquencés amphiphiles P spécifiques. Plus précisément, ces polymères séquencés amphiphiles P comprennent :
- un bloc hydrophile A contenant des groupements R^{A} susceptibles de développer des interactions avec la surface des particules ; et
- au moins un bloc hydrophobe B, lié au bloc hydrophile A, présentant une affinité vis-à-vis du milieu organique dispersant.

Les travaux des inventeurs ont maintenant permis de mettre en évidence que l'utilisation des polymères séquencés P tels que définis ci-dessus permet d'obtenir une stabilisation particulièrement efficace de la dispersion de particules dans l'organosol.

Par le terme de "dispersion stabilisée", on entend, au sens de la présente description, une dispersion de particules dans un milieu dispersant qui est de préférence un liquide de faible viscosité, typiquement présentant une viscosité propre inférieure ou égale à 200 centipoise, et où les particules sont réparties de façon homogène dans ledit milieu dispersant, et où cette répartition homogène des particules est sensiblement conservée, sans phénomène sensible de sédimentation des particules, après un stockage d'un mois à 20°C, et le plus souvent après un stockage d'au moins 6 mois, voire de 12 mois, à 20°C.

Les dispersions stabilisées de la présente invention présentent par ailleurs une bonne stabilité thermique, aussi bien à des températures importantes que dans des conditions de températures variant sur une grande amplitude.

Ainsi, on constate en général qu'à l'issue d'un traitement thermique isotherme à une température de l'ordre de 70°C pendant 1 mois, voire pendant 3 mois, les particules d'un organosol selon l'invention restent réparties de façon homogène, sans phénomène sensible de sédimentation des particules. Le plus souvent, le traitement thermique isotherme d'un organosol selon l'invention pendant un mois à une température de 80°C, voire de 90°C, ne conduit pas à une déstabilisation induisant des phénomènes de sédimentation sensible des particules.

De la même façon, on conserve également une bon maintien de la stabilité, sans phénomène sensible de sédimentation des particules, si on soumet un organosol selon l'invention à des cycles de températures entre -20°C et +90°C pendant au moins 100 heures, et le plus souvent pendant au moins 200 heures, voire plus.

Au sens où elle est utilisée dans la présente description, l'expression "sans phénomène sensible de sédimentation des particules" désigne une absence de sédimentation des particules détectable visuellement. La stabilité au stockage et l'inhibition des phénomènes de sédimentation qui sont obtenues avec les organosols de la présente invention peut être quantifiée de façon plus précise en comparant la quantité de particules présentes à l'état dispersé dans l'organosol initial et dans l'organosol à l'issue du stockage.

Dans la plupart des cas, on constate qu'après un stockage de l'organosol dans les conditions précitées, au moins 80, et le plus souvent au moins 90%, voire au moins 95% des particules initialement à l'état dispersées restent en dispersion, sans sédimenter.

La nature des particules minérales qui sont présentes dans les dispersions stabilisées de l'invention peut varier en une assez large mesure. Il s'agit toutefois avantageusement de particules à base d'un oxyde minéral. Il peut ainsi en particulier s'agir de particules à base de silice SiO₂, ou bien de particules à base d'un oxyde métallique, tel que l'oxyde de cérium CeO₂, l'oxyde de titane TiO₂, l'oxyde de zirconium ZrO₂, l'alumine Al₂O₃, un oxyde de Fer tel que Fe₂O₃, ou un mélange de deux ou plus de ces oxydes.

L'expression "particules à base d'un oxyde minéral" désigne ici des particules formées en tout ou partie par un oxyde minéral, de préférence choisi parmi les oxydes précités ou leurs mélanges. En général, dans une dispersion selon l'invention, les particules présentes sont majoritairement constituées par oxyde minéral ou par mélange d'oxydes minéraux,_ c'est-à-dire qu'elles comprennent de préférence au moins 50% en masse, plus avantageusement au moins 80% en masse, et plus préférentiellement au moins 90% en masse d'un ou plusieurs oxydes minéraux. Selon un mode de réalisation particulier, les particules présentes sont essentiellement constituées d'oxyde minéral (à savoir à raison d'au moins 90% en masse, de préférence au moins 95% en masse, plus préférentiellement au moins 99% en masse). Selon un autre mode de réalisation possible, les particules peuvent comprendre un coeur à base d'un premier oxyde, enrobé par une couche à base d'un autre oxyde. Il peut ainsi par exemple s'agir de particules à base de CeO₂ enrobées par une couche de silice.

Selon un mode de réalisation particulier, les oxydes présents dans les particules des organosols de l'invention peuvent être dopés par un ou plusieurs éléments métalliques. Un tel oxyde dopé peut être par exemple de la silice dopée par des cations aluminium, ou bien un oxyde d'un premier métal dopé par des cations d'un autre métal.

La teneur en particules au sein d'une dispersion selon l'invention peut varier en une assez large mesure, en fonction des applications envisagées pour cette dispersion. Toutefois, dans le cas le plus général, elle est le plus souvent comprise entre 0,1% et 15% en masse par rapport à la masse totale de la dispersion. Cette gamme permet en effet d'obtenir des organosols relativement peu visqueux, ayant une bonne fluidité et qui sont faciles à manipuler. Dans ce cadre, pour obtenir une viscosité la plus réduite possible, il est préférable que la teneur en soit inférieure ou égale à 13 % en masse, plus préférentiellement inférieure ou égale à 10% en masse, par exemple inférieure ou égale à 8% en masse par rapport à la masse totale de la dispersion. En général, on préfère toutefois que l'organosol ne soit pas trop dilué, et il s'avère souvent préférable que la teneur en particules reste d'au moins 0,2 % en masse, par exemple d'au moins 0,4 % en masse par rapport à la masse totale de la dispersion. Ainsi, une dispersion selon l'invention peut typiquement comprendre de l'ordre de 0,2% à 10% en masse de particules, par exemple entre 0,5 et 5% en masse, par rapport à la masse totale de la dispersion.

Selon un autre mode de réalisation envisageable, un organosol selon l'invention peut avoir une teneur en particules supérieure à 15%. Dans ce cas, l'organosol présente généralement une viscosité élevée, mais conserve néanmoins ses bonnes propriétés de stabilité au stockage.

Quelle que soit leur nature chimique exacte, les particules minérales contenues dans les dispersions stabilisées de l'invention sont, en règle générale, essentiellement présentes au sein de l'organosol sous la forme de particules individualisées et/ou d'agrégats de particules, qui sont dispersés dans le milieu organique dispersant. Chacun des objets ainsi dispersé est entouré par des polymères P, ce qui forme schématiquement dans l'organosol des espèces mixtes inorganiques/organiques comprenant (i) un coeur minéral à base d'une ou de quelques particules minérales et (ii) une couche organique à base des polymères P. Le coeur minéral de ces espèces a en général une taille moyenne inférieure à 1 µm, typiquement inférieure à 200 nm, et le plus souvent inférieure à 100 nm, abstraction faite de la couche organique l'entourant. Cette taille moyenne du coeur minéral des espèces dispersées dans les organosols de l'invention peut notamment être déterminée par analyse de clichés de microscopie électronique à transmission d'échantillons de l'organosol (par exemple réalisés selon la technique de l'ultracryotomie).

Les polymères séquencés amphiphiles P spécifiques qui sont utilisés à titre d'agents stabilisants dans le cadre de l'invention permettent de fournir des dispersions stables, où les objets dispersés sont souvent sous la forme de particules individualisées et/ou sous forme d'agrégats de quelques particules qui présentent en général des dimensions de l'ordre de grandeur des particules individualisés, et ce même lorsque les particules présentes sont de très faible taille, par exemple de l'ordre de quelques nanomètres ou de quelques dizaines de nanomètre. Les propriétés de stabilité conférées par les polymères P aux organosol comprenant des particules de cette gamme de taille se révèlent étonnamment élevée en comparaison des résultats observés lorsqu'on essaie de stabiliser le sol au moyen d'agents stabilisant usuels de type tensioactifs, tels que l'acide isostéarique par exemple. Ces propriétés de stabilisation des polymères P, mises en évidence par les inventeurs, permettent de fournir, dans le cadre de l'invention, des organosols qui comprennent des objets dispersés ayant de très faibles dimensions. Ainsi, les objets minéraux (particules individualisés ou agrégats minéraux) qui sont dispersés dans un organosol selon l'invention peuvent typiquement présenter une taille moyenne inférieure ou égale à 70 nm, voire à 60 nm. Ainsi, la taille moyenne des objets minéraux dispersés dans l'organosol est typiquement comprise entre 1 et 70 nm, typiquement entre 2 et 60 nm, par exemple entre 3 et 50 nm. La taille moyenne des objets minéraux à laquelle il est fait référence ici est celle des objets minéraux eux-mêmes, sans prendre en considération la couche d'espèces organiques (polymères P et éventuelle couche de solvatation) qui entoure ces objets minéraux.

Les dispersions stabilisées de la présente invention sont de façon spécifique, des dispersions d'objets minéraux stabilisés au sein d'un milieu organique. La mise en oeuvre spécifique des polymères amphiphiles P de l'invention confère une très grande liberté dans le choix du milieu organique dispersant utilisable. Dans ce cadre, la plupart des milieux organiques se révèlent en fait envisageables, sous réserve de choisir un polymère P comprenant un bloc hydrophobe B adapté au milieu employé.

Il est en particulier à souligner que la mise en oeuvre des polymères P selon l'invention autorise la mise en oeuvre de milieux organiques dispersants aussi bien apolaires que polaires, Par "milieu polaire", on entend ici un milieu ayant une constante diélectrique εᵣ supérieure à 5, la constante diélectrique εᵣ à laquelle il est fait référence ici étant telle que définie notamment dans l'ouvrage "Solvents and Solvent Effects in Organic Chemistry", C. Reichardt, VCH, 1988. A titre d'exemple de milieux polaires au sens de la présente description, on peut notamment citer la plupart des esters tels que, par exemple, l'acétate d'éthyle, le palmitate d'isopropyle ou bien encore l'acétate de méthoxypropyle; les composés halogénés tels que le dichlorométhane; les alcools comme l'éthanol, le butanol ou l'isopropanol ; les polyols tels que le propanediol, le butanediol ou le diéthylèneglycol ; ou bien encore les cétones comme la cyclohexanone ou la 1-méthylpyrrolidin-2 one.

De façon plus générale, le milieu organique dispersant des organosols de l'invention peut varier en une très large mesure. A titre indicatif, ce milieu organique dispersant peut par exemple comprendre un ou plusieurs composés organiques choisis parmi :
- les hydrocarbures aliphatiques ou cycloaliphatiques, tels que l'hexane, l'heptane, l'octane, le nonane, le décane, le cyclohexane, le cyclopentane, le cycloheptane ;
- les solvants aromatiques tels que le benzène, le toluène, l'éthylbenzène et les xylènes ;
- les mélanges d'hydrocarbures aromatiques et/ou aliphatiques, tels que les essences minérales ou les essences de pétrole, les naphtènes liquides, ou les coupes pétrolière du type ISOPAR ou SOLVESSO (marques déposées par la société EXXON), tels que le SOLVESSO 100 (mélange à base de méthyléthyl et triméthylbenzène) et le SOLVESSO 150 (mélange d'alkylbenzènes contenant en particulier du diméthyléthybenzène et du tétraméthylbenzène) ;
- les hydrocarbures chlorés tels que, par exemple, le chlorobenzène ou le dichlorobenzène, ou bien encore le chlorotoluène ;
- les éthers aliphatiques et cycloaliphatiques, tels que l'éther de diisopropyle, ou l'éther de dibutyle ;
- les cétones aliphatiques et cycloaliphatiques, telles que la méthylisobutylcétone, la diisobutylcétone, l'oxyde de mésityle, ou bien encore l'acétone ;
- les aldéhydes ;
- les solvants azotés comme l'acétonitrile ;
- les alcools comportant de préférence de 1 à 10 atomes de carbone, tels que l'éthanol, le propanol, ou bien le butanol ;
- les phénols ;
- les esters, et en particulier :
   - les esters issus de la réaction d'acides carboxyliques comportant de préférence de 10 à 40 atomes de carbone (tels que les acides gras de tallol, d'huile de coco, de soja, de suif, d'huile de lin, l'acide oléique, l'acide linoléique, l'acide stéarique et ses isomères, l'acide pélargonique, l'acide caprique, l'acide laurique, l'acide myristique, l'acide dodécylbenzènesulfonique, l'acide éthyl-2 hexanoïque, l'acide naphténique, l'acide palmitique, ou l'acide hexoïque) avec des alcools comportant par exemple de 1 à 8 atomes de carbone, ces alcools étant généralement des alcools primaires ou secondaires (comme l'isopropanol) ou bien encore des glycols comme le glycérol ; et les mélanges de tels esters,;
   - les esters sulfoniques ou phosphoniques, issus de préférence de la réaction d'alcools comportant avantageusement de 1 à 8 atomes de carbone avec des acides sulfoniques aliphatiques, des acides phosphoniques aliphatiques, des acides alcoylarylsulfoniques, ou des acides alcoylarylphosphoniques possédant de préférence de l'ordre de 10 à 40 atomes de carbone, tels que l'acide toluène-sulfonique, l'acide toluène-phosphonique, l'acide lauryl-sulfonique, l'acide lauryl-phosphonique, l'acide palmityl-sulfonique, et l'acide palmityl-phosphonique.
- les monomères polymérisables tels que le styrène et ses dérivés, les acrylates d'alkyle, les methacrylate d'alkyle, ou bien encore les esters vinyliques ; ainsi que les mélanges de tels monomères ;
- les huiles de silicone ;
- les huiles essentielles ;
- les huiles organiques telles que les huiles végétales ; et
- les mélanges des composés précités.

Quelle que soit la nature des particules utilisées et du milieu dispersant mis en oeuvre, une suspension selon l'invention contient de façon caractéristique, des copolymères séquencés amphiphiles P tels que définis plus haut, assurant un rôle d'agents stabilisants. Ces polymères P suffisent généralement à assurer, à eux seuls, la stabilité recherchée pour suspension, et ils peuvent de ce fait être utilisés à titre d'uniques agents stabilisants dans la dispersion, à l'exclusion par exemple d'autres agents tels que des agents tensioactifs. Néanmoins, selon un mode de réalisation de l'invention envisageable, on peut utiliser les polymères P en association avec d'autres agents stabilisants connus, par exemple des agents tensioactifs tels que de l'acide isostéarique, ou bien des agents tensioactifs anioniques ou cationiques tels que par exemple le bromure de diméthyl-didodécyl-ammonium ou le dodécylbenzenesulfonate de sodium. Dans certains cas, ce type d'association peut permettre une stabilisation particulièrement efficace.

De même, il n'est pas non plus requis, selon l'invention, que les particules soient modifiées par greffage sur leur surface de groupements liés de façon covalente.

De façon générale, le choix des polymères P à utiliser dans une dispersion selon l'invention, dépend en particulier :
- de la nature des particules utilisées, qui détermine en particulier les groupements R^{A} qui peuvent être présents sur le bloc A ; et
- de la nature du milieu dispersant, vis-à-vis duquel au moins un bloc hydrophobe B présent sur le polymère P doit présenter une affinité.

Ainsi, les polymères P comportent spécifiquement des groupements R^{A} qui sont susceptibles de développer des interactions avec la surface des particules de l'organosol, et dont au moins une partie développe effectivement des interactions avec la surface des particules.

Dans ce cadre, il est avantageux (bien que non requis, dans le cas général) que les groupements R^{A} soient des groupes développant, ou tout au moins susceptibles de développer, des interactions de type liaisons ioniques, complexantes ou électrostatiques, avec la surface des particules. A cet effet, il est préférable d'utiliser des particules présentant en surface des espèces chargées électriquement (espèces cationiques ou anioniques) et des polymères P comprenant, à titre de groupements R^{A}, des groupements ayant une charge de signe opposé à celle des espèces présentes sur la surface des particules (groupements anioniques ou cationiques, respectivement).

Selon une première variante de l'invention, les particules minérales utilisées ont une surface chargée négativement et tout ou partie des groupements R^{A} du bloc A des polymères P sont des groupements de nature cationique. Ces groupes cationiques R^{A} sont en général associés avec des contre-ions chargés négativement, qui peuvent notamment être choisis parmi les ions chlorures, bromures, iodures, fluorures, sulfates, methylsulfates, phosphates, hydrogénophosphates, phosphonates, carbonates, et hydrogenocarbonates. De préférence ces contre-ions sont des hydrogenophosphates, des methylsulfates et/ou des chlorures.

Selon cette variante particulière de l'invention, les particules ayant une surface chargée négativement sont typiquement des particules à base de silice, et les groupement R^{A} du bloc hydrophile A des polymères P comprennent quant à eux le plus souvent des groupements ammonium ou amines à l'état quaternisées. Dans ce cadre, le bloc A peut typiquement être un bloc de type polyamine, poyethylenimine, polyallylamine, ou bien encore de type poly(acrylate de diméthylaminoéthyle), quaternisé ou non. Le bloc A peut alternativement être un bloc polymère greffé par des groupements cationiques après sa polymérisation. Par exemple, le bloc A peut être un bloc halogéné (tel qu'un bloc poly-p-chloromethylstyrène) sur lequel on fait réagir, après polymérisation, une amine tertiaire (triméthylamine, par exemple).

Selon un mode de réalisation intéressant dans le cas où les particules minérales utilisées ont une surface chargée négativement, le bloc hydrophile A des polymères P est un homopolymère ou un copolymère à base de monomères M dont au moins une partie comprend une insaturation éthylénique et au moins un atome d'azote quaternaire ou quaternisable par protonation (c'est-à-dire par adaptation du pH). Dans ce cadre, le bloc hydrophile A peut par exemple être à base d'un ou plusieurs des monomères suivants :
- les monomères de formule (M1) : 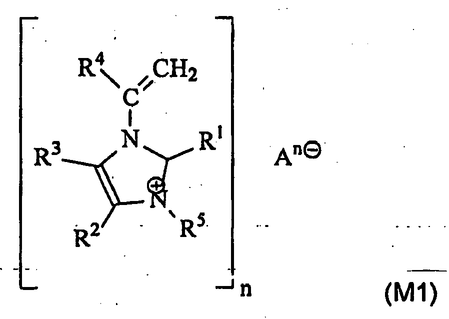 dans laquelle :
   A^{nΘ} représente un anion Cl^{Θ}, Br^{Θ}, I^{Θ}, SO₄^{2Θ}, CO₃^{2Θ}, CH₃-OSO₃^{Θ} ou CH₃-CH₂-OSO₃^{Θ},
   - les groupes R¹ à R⁵, identiques ou différents, représentent, indépendamment les uns des autres, un groupe alkyle ayant de 1 à 20 atomes de carbone, un radical benzyle ou bien un atome d'hydrogène ; et
   - n vaut 1 ou 2 ;
- les monomères de formule (M2): 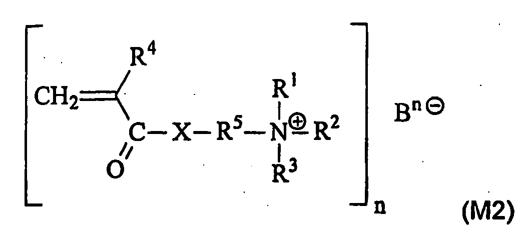 dans laquelle :
   - X représente un groupe -NH- ou un atome d'oxygène -O- ;
   - R4 représente un atome d'hydrogène, ou un groupe alkyle ayant de 1 à 20 atomes de carbone ;
   - R5 représente un groupe alcényle ayant de 1 à 20 atomes de carbone ;
   - les groupes R1, R2, R3, identiques ou différents, représentent, indépendamment les uns des autres, un groupe alkyle ayant de 1 à 20 atomes de carbone ;
   - B^{nΘ} représente un ion Cl^{Θ}, Br^{Θ}, I^{Θ}, SO₄^{2Θ}, CO₃^{2Θ}, CH₃-OSO₃^{Θ} ou CH₃-CH₂-OSO3^{Θ}; et
   - n vaut 1 ou 2 ;
- les monomères de formule (M3): 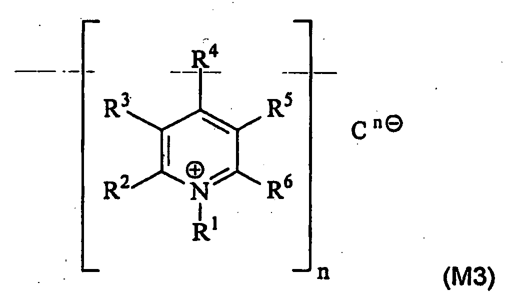 dans laquelle :
   - un des groupes R¹ à R⁶ représentant un groupe -CH=CH2, les autres groupes R¹ à R⁶, identiques ou différents, représentant, indépendamment les uns des autres, un atome d'hydrogène ou un groupe alkyle ayant de 1 à 20 atomes de carbone,
   - C^{nΘ} représente un ion Cl^{Θ}, Br^{Θ}, I^{Θ}, SO₄^{2Θ}, CO₃^{2Θ}, CH₃-OSO₃^{Θ} ou CH₃-CH₂-OSO₃^{Θ}; et
   - n vaut 1 ou 2, ou
- les monomères de formule (M4): 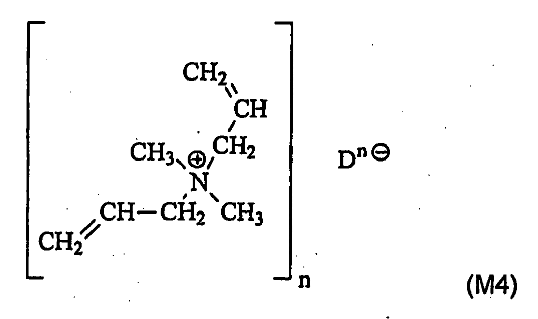 dans laquelle :
   - D^{nΘ} représente un ion Cl^{Θ}, Br^{Θ}, I^{Θ}, SO₄^{2Θ}, CO₃^{2Θ}, CH₃-OSO₃^{Θ} ou CH₃-CH₂-OSO₃^{Θ}; et
   - n vaut 1 ou 2.

De préférence, lorsque les particules minérales utilisées ont une surface chargée négativement, le bloc hydrophile A comprend, à titre de monomères M, des monomères choisis parmi :
- l'acrylate de 2-dimethylaminoethyle (ADAM) ;
- le méthacrylate de 2-dimethylaminoethyle (MADAM) ; ou le
- le chlorure de diallyldimethylammonium (DADMAC).

Selon une autre variante de l'invention, les particules minérales présentes dans l'organosol ont une surface chargée positivement, et tout ou partie des groupements R^{A} du bloc A des polymères P sont des groupements de nature anionique. Dans le cadre de ce mode de réalisation spécifique, les particules peuvent notamment être des particules à base d'oxyde de cérium CeO₂, d'oxyde de titane TiO₂, ou d'oxyde de zirconium ZrO₂, et le bloc A des polymères P comporte avantageusement des groupements carboxylate (-COO⁻), sulfate (-SO₄⁻), sulfonate (-SO₃⁻), phosphonate (par exemple sous la forme-PO₃²⁻) ou phosphate (par exemple sous la forme -OPO₃²⁻). De façon particulièrement avantageuse, selon ce mode de réalisation, les particules minérales sont des particules à base d'oxyde de cérium CeO₂. En particulier dans ce cas, il est avantageux que le bloc A comporte des groupements -COOH libres, de préférence au moins partiellement ionisés à l'état de carboxylates (-COO⁻). Dans ce cadre, le bloc A est avantageusement un bloc polyacrylate ou poly(acide acrylique), ou bien un bloc poly(styrène sulfonate), un bloc poly(acide vinylphosphonique) ou bien encore un bloc poly(acide acrylamido methylpropanesulfonique).

Dans le cas le plus général, quelle que soit la nature des particules minérales et leur charge de surface, il peut se révéler avantageux que le bloc hydrophile A des polymères P comprenne, en plus les groupes R^{A}, des unités hydrophiles issues de l'incorporation de monomères hydrophiles dans ce bloc A. Par « monomère hydrophile », on entend ici des monomères dont la solubilité dans l'eau est supérieure à 52g/l à 20°C sous une pression de 1 bar. A titre d'exemple de tels monomères hydrophiles, on peut notamment citer hydroxyéthy-lacrylates, les hydroxyéthyl-methacrylates, l'acrylamide, la N-vinylpyrrolidone, ou bien encore des macromonomères tels que les acrylates ou les methacrylates de polyoxyde d'éthylène et/ou de propylène, ou l'alcool polyvinylique.

D'autre part, indépendamment de la nature du bloc A et des groupements R^{A}, le ou les bloc(s) B présent(s) dans les polymères P sont des blocs présentant une affinité vis-à-vis du milieu organique dispersant. Par "bloc B présentant une affinité vis-à-vis du milieu dispersant", on entend au sens de la présente description un bloc qui est soluble dans le milieu dispersant, lorsqu'il est pris à l'état isolé (à savoir sous la forme d'un polymère B, isolé du bloc A du polymère P).

Pour choisir un bloc B adapté à un milieu dispersant particulier, on pourra ainsi choisir un bloc dont les paramètres de solubilité sont compatibles avec ceux du milieu dispersant. Par "paramètres de solubilité", on entend ici les paramètres tels que définis notamment dans l'ouvrage "Handbook of solubility parameters and other cohesion parameters"de Allan FM BARTON, CRC Press, Boca Raton (Floride), ISBN 0-8493-3295-8. On pourra également se reporter à cet ouvrage pour ce qui est du calcul de ces paramètres de solubilité et leur utilisation pour déterminer la compatibilité d'un soluté tel qu'un polymère dans un solvant donné.

Le Tableau I ci-dessous donne, à titre indicatif, des exemples de blocs polymères utiles à titre de bloc B pour des solvants de différentes polarités. Les exemples donnés dans ce tableau sont uniquement donnés à titre informatif, et l'invention ne saurait naturellement se limiter à ces exemples spécifiques de couples (bloc B/milieu dispersant).

**Tableau I :**

| Exemples de blocs B adaptés pour certains milieux dispersants particuliers | |
|---|---|
| Milieu dispersant utilisé | Exemple de Bloc B adapté |
| acétate de butyle | poly(acrylate de 2-éthyl-hexyle) |
| | Poly(acrylate d'isooctyle) |
| | Poly(acrylate de butyle) |
| | Polymère statistique ⁽¹⁾ (acrylate de butyle / acrylate de 2-hydroxyethyle) |
| Exxsol D40 | poly(acrylate de 2-éthyl-hexyle) |
| | Poly(acrylate d'isooctyle) |
| | Poly(acrylate de butyle) |
| | Polymère statistique ⁽²⁾acrylate de butyle / acrylate de 2-hydroxyethyle |
| Isopar L | Poly(acrylate de 2-ethyl hexyle) |
| Mélange de méthacrylate de méthyle et d'acrylate de 2-ethyl hexyle (50/50 en poids) | Poly(acrylate de 2-ethyl hexyle) |
| Cyclométhicone | Poly(diméthylsiloxane) |

| | |
|---|---|
| ⁽¹⁾ : un bloc polymère adapté comprend par exemple de 10% à 30% en poids d'acrylate de 2-hydroxyéthyle (notamment 13% ou 26% en poids). ⁽²⁾ : un bloc polymère adapté comprend par exemple de 10 à 20% en poids d'acrylate de 2-hydroxyéthyle (notamment 13% en poids). | |

Les polymères P utilisés à titre d'agents de stabilisation dans l'organosol de la présente invention peuvent présenter différentes structures, indépendamment de la nature chimique exacte de leurs blocs A et B.

Ainsi, selon une première variante, les polymères P utilisés à titre d'agents de stabilisation de la dispersion comprennent (voire sont essentiellement) des polymères diblocs, constitués d'une association du bloc hydrophile A et du bloc hydrophobe B, à savoir des polymères de formule schématique A-B.

Selon une autre variante, les polymères P utilisés à titre d'agents de stabilisation de la dispersion comprennent (voire sont essentiellement) des copolymères ayant plusieurs blocs hydrophobes (B1, B2, ...BN, où N peut aller de 2 à 100) liés de façon covalente au bloc hydrophile A. Les copolymères à base de plusieurs blocs hydrophobes selon cette variante comprennent avantageusement (et de préférence sont) :
- des polymères séquencés de type tribloc, de formule B1-A-B2 où chacun des groupements B1 et B2 représente un bloc hydrophobe de type B précité ; et/ou
- des polymères de type peigne dans lesquels plusieurs blocs hydrophobes de type B précité sont liés à titre de chaînes latérales, sur le bloc hydrophile A. Typiquement, ces polymères de type peigne ont la formule schématique ci-après :

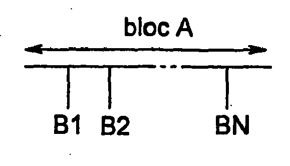

Quelle que soit la structure exacte du polymère P, il est le plus souvent avantageux que les polymères P comportent statistiquement un nombre moyen de groupement R^{A} supérieur à 1 au sein du bloc A ce nombre moyen de groupements R^{A} au sein du bloc A étant de préférence au moins égal à 2, par exemple entre 2 et 4, ce nombre moyen restant en général inférieur à 7, avantageusement inférieur ou égal à 5. En effet, les travaux des inventeurs montrent que, plus le nombre de groupements R^{A} est important dans le bloc A, plus l'effet de stabilisation augmente. Sans vouloir être lié à une quelconque théorie, il semble que l'augmentation du nombre de groupements R^{A} dans le bloc A induit une multiplication des probabilités de liaison entre les particules et les polymères P, ce qui induit une augmentation de la solidarisation des deux espèces, ce qui n'est pas observés avec des agents tensioactifs classiques de type acide oléique ou acide isostéarique.

Par ailleurs, notamment pour limiter la viscosité du sol et éviter une teneur massique trop importante en polymère P dans l'organosol, on préfère en général que le polymère P utilisé ait une masse moléculaire inférieure à 10 000 g/mol, cette masse moléculaire étant de préférence inférieure à 5 000 g/mol, et avantageusement inférieure ou égale à 3 000 g/mol. Néanmoins, les polymères utilisés ont en général une masse moléculaire plus élevée que celle des agents tensioactifs classiquement utilisés pour la stabilisation d'organosols. Ainsi, la masse moléculaire des polymères P mis en oeuvre selon l'invention est de préférence supérieure ou égale à 1000 g/mol, et elle est avantageusement comprise entre 1 000 et 3 000 g/mol, par exemple entre 1 500 et 2 500 g/mol..

D'autre part, dans les polymères P, le rapport massique (bloc hydrophile A / bloc(s) B) est avantageusement compris entre 0,02 et 0,5. Ce rapport correspond en général au rapport dit "balance hydrophile/lipophile" (rapport "HLB") du polymère. Le rapport massique (bloc hydrophile A / bloc(s) B) a mettre en oeuvre dépend pour l'essentiel de la concentration en particules dans la dispersion et de la nature dispersant et sa valeur optimale est de ce fait à adapter au cas par cas. Néanmoins, dans la plupart des cas, de bonnes propriétés de stabilisation sont obtenues pour des rapports massiques (bloc hydrophile A / bloc(s) B) dans les polymères P compris entre 0,02 et 0,5, et en particulier lorsque ce rapport est compris entre 0,05 et 0,2.

Par ailleurs, dans un polymère P utilisé selon l'invention, la masse moléculaire du bloc A est de préférence comprise entre 50 et 5000 g/mol, et plus avantageusement entre 100 et 1000 g/mol. La masse moléculaire de chacun des groupements hydrophobes B présents dans les polymères P est quant à elle généralement plus importante que celle du bloc A, cette masse moléculaire étant avantageusement comprise entre 500 et 8000 g/mol, par exemple entre 1000 et 4000 g/mol.

Dans le cas le plus général, quelle que soit la nature et la structure des polymères P et des particules présentes, le ratio massique (particules/polymère) au sein d'une dispersion selon l'invention est de préférence supérieur ou égal à 0,1, de préférence supérieur ou égal à 0,2. Typiquement, ce ratio massique (particules/polymère) au sein de la dispersion est compris entre 0,2 et 0,6, par exemple entre 0,2 et 0,5.

Par ailleurs, on préfère en général que le ratio molaire (groupes R^{A} du bloc hydrophile A / constituant minéral des particules), soit supérieur ou égal à 0,2, et de préférence supérieur ou égal à 0,3, ce rapport étant avantageusement compris entre 0,3 et 1, typiquement entre 0,4 et 0,8, avantageusement entre 0,4 et 0,6.

D'autre part, il est souvent avantageux que, dans une dispersion selon l'invention le ratio massique (polymères / solvant) soit supérieur ou égal à 0,005, et de préférence d'au moins 0,02, ce qui permet d'obtenir des effets de stabilisation marqués. Notamment pour éviter une augmentation trop importante de la viscosité de la suspension, on préfère toutefois que ce rapport reste inférieur à 0,7, et de préférence inférieur à 0,5.

A titre de polymères P particulièrement adaptés dans le cadre de la présente invention, on peut notamment utiliser des polymères séquencés tels qu'obtenus notamment selon les procédés de polymérisation radicalaire contrôlée (vivante) décrits par exemple dans les demandes de brevets WO 98/58974, WO 00/75207 et WO 01/4231.

La mise en oeuvre des procédés de polymérisation radicalaire vivante permet d'obtenir des polymères P de structure et de taille particulièrement bien définie et dont la fonctionnalité et les propriétés de chacun des blocs peuvent être maîtrisées et modulées de façon très fine. Ces procédés permettent ainsi, entres autres avantages, d'obtenir des polymères P porteurs de fonctions réactives (des groupements OH par exemple), ce qui permet d'obtenir des organosols stabilisés selon l'invention, dans lesquels les polymères ne se cantonnent pas à assurer un rôle d'agents de stabilisation, mais peuvent fournir d'autres effets. De plus, la mise en oeuvre des procédés des demandes de brevets WO 98/58974, WO 00/75207 et WO 01/4231 permet dans ce cadre une polymérisation de monomères ioniques et porteurs de groupements anioniques ou cationiques dans des conditions douces, sans nécessiter une protection des groupes ioniques.

Des polymères séquencés P particulièrement intéressants sont ceux susceptibles d'être obtenus selon un procédé de polymérisation radicalaire contrôlé comprenant les étapes successives suivantes :
(e1) on forme un premier bloc polymère fonctionnalisé en bout de chaîne en mettant en contact :
   - au moins un monomère éthyléniquement insaturé,
   - au moins une source de radicaux libres, et
   - au moins un xanthate, un dithioester, une thioether-thione, un dithiocarbazate, ou un dithiocarbamate, de préférence un xanthate, et avantageusement un composé du type de ceux décrits dans la demande WO 01/4231 ; puis
(e2) on forme sur le premier bloc polymère fonctionnalisé en bout de chaîne obtenu dans l'étape (e1) un deuxième bloc, en faisant réagir le polymère obtenu avec un nouveau monomère éthyléniquement insaturé et une source de radicaux libres.

Ce procédé de préparation peut avantageusement être utilisé pour la synthèse de polymères séquencés P de type dibloc.

Dans ce cadre, selon un premier mode de réalisation, on forme dans l'étape (e1) le bloc hydrophile du polymère dibloc (par exemple un bloc poly(acide acrylique)) et, dans l'étape (e2) on fait croître sur le bloc hydrophile obtenu un deuxième bloc, cette fois-ci de nature hydrophobe.

A l'inverse, selon un autre mode de réalisation, on peut former dans l'étape (e1) le bloc hydrophobe du polymère dibloc (par exemple un bloc poly(acide acrylique)). Dans ce cas, l'étape (e2) est conduite de façon à faire croître sur le bloc hydrophobe obtenu un deuxième bloc de nature hydrophile.

Il est à souligner que; pour un polymère dibloc de composition donnée, ces deux modes de préparation ne s'avèrent pas équivalents, et conduisent en pratique à des polymères qui présentent souvent des propriétés de stabilisation d'organosols différentes, et ce en particulier lorsque le bloc hydrophile est de très faible taille. Souvent, il s'avère préférable de réaliser tout d'abord la synthèse du bloc hydrophile dans l'étape (e1), puis celle du bloc hydrophobe dans l'étape (e2).

Selon un autre mode de réalisation particulier, l'étape (e2) peut être effectuée deux fois de suite pour synthétiser des polymères (P) de type tribloc. Dans ce cas, on synthétise généralement un premier bloc hydrophobe dans l'étape (e1), sur lequel on fait croître un bloc hydrophile, puis on fait croître à l'extrémité du bloc hydrophile ainsi obtenu un nouveau bloc hydrophobe.

De façon générale, il est préférable, dans le cadre de la présente invention, de conduire les étapes (e1) et (e2) en utilisant des composés de type xanthate, et de préférence des xanthates porteurs de groupements O-ethyle ou O-trifluoroethyle. Par ailleurs, les étapes (e1) et (e2) sont avantageusement conduites en solution.

Lorsque les particules de la dispersion de l'invention ont une charge de surface négative (particules à base de silice notamment), ont peut ainsi notamment utiliser à titre de polymères P des polymères issus des procédés précédemment décrits et porteurs de groupement cationiques (ammonium quaternaires) sur leur bloc hydrophile, tels que des polymères séquencés de type poly(acrylate de butyle)-poly(acrylate de 2-dimethylaminoethyle quaternisé) ; poly(acrylate de 2-ethylhexyle)-poly(acrylate de 2-dimethylaminoethyle quaternisé) ; ou poly(acrylate de 2-ethylhexyle)-poly(p-chloromethyl styrène quaternisé).

A l'inverse, lorsque les particules de la dispersion de l'invention ont une charge de surface positive (particules à base de CeO₂ ou TiO₂ notamment), ont peut notamment utiliser à titre de polymères P des polymères issus des procédés précédents et porteurs de groupement anioniques (carboxylates, sulfates ou sulfonates par exemple) sur leur bloc hydrophile, tels que des polymères séquencés de type poly(acrylate de butyle)-poly(acide acrylique) ; poly(acrylate de 2-ethylhexyle)-poly(acide acrylique), poly(acrylate de 2-ethylhexyle)-poly(styrène sulfonate); poly(acrylate de butyle)-poly(styrène sulfonate) ; poly(acrylate d'isooctyle)-poly(acide acrylique) ; ou poly(acrylate de 2-ethyl hexyle)-poly(acide vinyl phosphonique).

Lorsque les particules de la dispersion de l'invention ont une charge de surface positive, et tout particulièrement lorsqu'il s'agit de particules à base d'oxyde de cérium, il s'avère avantageux d'utiliser, à titre de polymères P, des polymères poly(acide acrylique)-poly(2-ethylhexylacrylate) (dits ici PAA-P2EHA), et avantageusement les polymères de ce type ayant une masse molaire de 1500 à 4000 g/mol et où le rapport massique (PAA/ P2EHA) est de préférence de 0,02 à 0,5 (avantageusement de 0,05 et 0,2).

Les inventeurs ont en effet mis en évidence que ces copolymères PAA-P2EHA particuliers se révèlent adaptés à la stabilisation de la dispersion de particules à charge de surface positive (particules de CeO2 par exemple) dans la plupart des milieux organiques dispersants utilisés dans le domaine des organosols. En d'autres termes, les polymères PAA-P2EHA précités constituent en quelque sorte un "stabilisant universel" pour la dispersion de particules de types particules à base d'oxyde de cérium dans l'essentiel des solvants organiques utilisables à titre de milieux dispersants.

Avantageusement, les polymères PAA-P2EHA utilisés dans ce cadre contiennent un bloc hydrophile polyacide acrylique (PAA) de masse moléculaire de l'ordre de 125 à 150 g/mol, et un bloc hydrophobe poly(2-ethylhexylacrylate) (P2EHA) ayant une masse moléculaire de l'ordre de 1000 à 9000 g/mol (par exemple de l'ordre de 2250 g/mol).

Les polymères PAA-P2EHA précités constituent, selon un aspect spécifique, un objet particulier de la présente invention..

Selon un autre aspect, la présente invention a également pour objet la préparation des organosols décrits précédemment.

Dans ce cadre, l'invention fournit d'abord, selon un premier aspect particulier, un procédé qui permet la préparation des dispersions du type précitées où le milieu organique dispersant est spécifiquement de nature hydrophobe. Ce procédé comprend une étape (A) consistant à mettre en contact une suspension aqueuse des particules avec ledit milieu organique, en présence des polymères séquencés amphiphiles P tels que définis précédemment, ce par quoi on obtient un transfert des particules de la phase aqueuse vers le milieu solvant hydrophobe sous la forme d'une suspension organique stabilisée par les polymères.

En général, cette étape (A) est suivie par la récupération de la phase organique qui constitue la dispersion recherchée (organosol).

Dans le cas le plus général, l'étape (A) est en fait analogue aux procédés de transfert de phase classiquement utilisés pour réaliser des organosols stabilisés par des tensioactifs, de type acide oléique ou acide isostéarique par example, tels que ceux décrits par example dans la demande de brevet FR 2 721 615 ou dans l'article de Meriguet et coll. dans le Journal of Colloid and Interface Science, 267, pp. 78-85 (2003). En règle générale, les conditions définies pour la mise en oeuvre d'agents tensioactifs peuvent être directement transposées à la mise en oeuvre de polymères P utilisés selon la présente invention.

De façon plus spécifique, dans le cadre de la présente invention, la suspension aqueuse initiale utilisée dans l'étape (A) a avantageusement une concentration en particules minérale comprise entre 1 et 100 g/L. En termes de transfert de phase, l'étape (A) se révèle le plus souvent d'autant plus efficace, que cette concentration est faible. De ce fait, on préfère en général utiliser une concentration en particules minérale inférieure ou égale à 75 g/L, par exemple inférieure ou égale à 50 g/L, de préférence encore inférieure ou égale à 40 g/L et encore plus avantageusement inférieure à 20 g/L, dans la suspension aqueuse initiale de l'étape (A). Toutefois, notamment d'un point de vue économique et en termes de traitement d'effluents, il est le plus souvent souhaitable que cette concentration soit d'au moins 2 g/l, de préférence d'au moins 3 g/L, et plus préférentiellement d'au moins 5 g/L. Ainsi, cette concentration est typiquement comprise entre 2 et 75 g/L, avantageusement entre 3 et 50 g/L, et typiquement de l'ordre de 5 à 30 g/L, par exemple de l'ordre de 20 g/L.

La suspension aqueuse initialement mise en oeuvre dans l'étape (A) contient des particules à l'état dispersé qui, pour l'essentiel au moins, vont se retrouver à l'état dispersés dans la phase organique, à l'issue de l'étape (A). Les dispersions aqueuses utilisées dans ce cadre comprennent avantageusement des particules ayant un faible diamètre hydrodynamique au sein de la dispersion aqueuse, par exemple un diamètre hydrodynamique moyen compris entre 1 et 200 nm, ce diamètre hydrodynamique étant de préférence inférieur à 70 nm, et plus avantageusement inférieur ou égal à 50 nm. Le "diamètre hydrodynamique moyen" des particules auquel il est fait référence ici est celui déterminé selon la méthode de diffusion quasi-élastique de la lumière, telles que décrite notamment dans Analytical Chemistry, vol. 53, n° 8, 1007 A, (1981). Typiquement, le diamètre hydrodynamique moyen des particules dans la dispersion aqueuse initiale de l'étape (A) est comprise entre 1 et 70 nm, par exemple entre 2 et 60 nm, notamment entre 3 et 50 nm

Le procédé de l'invention permet de fournir des organosols à partir de dispersions aqueuses de particules ayant de très faibles dimensions, par exemple ayant un diamètre hydrodynamique moyen inférieur ou égal à 50 nm, voire inférieur ou égal à 20 nm, ou même inférieur ou égal à 10 nm, par exemple de l'ordre de quelques nanomètres. Dans ce cadre, compte tenu de la spécificité du procédé et de l'emploi des polymères P, le procédé de l'invention conduit le plus souvent à l'obtention d'organosols où la taille des objets minéraux dispersés (taille mesurable par microscopie électronique de l'organosol, ne tenant pas compte de la couche organique qui entoure ces objets minéraux) est de l'ordre de grandeur du diamètre hydrodynamique des particules dispersées dans la suspension aqueuse initiales. Ainsi, en partant de sols aqueux de particules de faibles dimensions, on obtient selon le procédé de l'invention des organosols à base d'objets minéraux dispersés de faibles tailles également. A l'inverse, on peut, si on le souhaite, obtenir des objets dispersés de taille plus importante dans l'organosol final, en utilisant dans l'étape (A) une suspension aqueuse où le diamètre hydrodynamique moyen des particules est plus élevé.

Par ailleurs, on préfère utiliser dans l'étape (A) des suspensions aqueuses où les particules ont une surface spécifique BET comprise entre 5 et 500 m²/g, de préférence d'au moins 50 m²/g, et plus préférentiellement d'au moins 100 m²/g, par exemple d'au moins 200 m²/g et notamment d'au moins 250 m²/g, pour lesquelles on obtient une bonne efficacité de transfert du milieu aqueux au milieu organique. Par "surface spécifique BET", on entend ici la surface spécifique telle que déterminée par adsorption d'azote conformément à la norme ASTMD 3663-78 établie à selon la méthode BRUNAUER - EMMETT - TELLER décrite dans "The Journal of the American Society", 60, 309 (1938). Pour mesurer la surface spécifique des particules selon l'invention, lorsqu'elles se présentent sous forme de dispersion, le protocole de mesure comprend une élimination de la phase liquide de la dispersion puis un séchage des particules sous vide à une température de 150°C pendant au moins 4 heures.

Le procédé de l'invention est fondé sur un transfert des particules du milieu aqueux vers le milieu organique. A ce sujet, il est à noter qu'outre leur propriétés de stabilisation de l'organosol sol final avec maintien d'un bon état de dispersion des objets minéraux, les copolymères séquencés P tels qu'utilisés selon l'invention permettent en outre de réaliser le transfert des particules avec une efficacité et un rendement important. Ainsi, dans le cadre du procédé de l'invention, on obtient en règle générale des rendements de transfert des particules du milieu aqueux vers le milieu organique d'au moins 80%, et qui sont le plus souvent d'au moins 90%, voir d'au moins 95%.

Pour optimiser l'efficacité de ce transfert est obtenir des rendements importants, on peut en outre agir notamment sur les différents paramètres suivants du procédé :
- le nombre moyen de groupements R^{A} au sein du bloc A des polymères P :
   dans ce cadre, les travaux des inventeurs ont permis d'établir que le rendement du transfert de phase est d'autant plus élevé que le nombre moyen de groupements R^{A} au sein du bloc A est important. Pour obtenir de bons rendements, ce nombre moyen est de préférence au moins égal à 2, et avantageusement au moins égal à 3.
- la masse moléculaire des polymères P :
   le rendement de transfert a généralement tendance à chuter lorsque cette masse augmente, notamment en raison de problèmes de diffusions observés avec les polymères lorsque leur taille augment. Pour obtenir des rendements de transferts intéressants, il est le plus souvent indiqué d'utiliser des polymères ayant une masse moléculaire inférieure à 10 000 g/mol, de préférence inférieure à 5 000 g/mol, et avantageusement inférieure ou égale à 3 000 g/mol, par exemple comprise entre 1 000 et 3 000 g/mol, et typiquement entre 1 500 et 2 500 g/mol.
- le rapport massique (bloc hydrophile A / bloc(s) B) dans les polymères P
   Dans la plupart des cas, de bons transferts sont obtenues pour un rapport massique (bloc hydrophile A / bloc(s) B) compris entre 0,02 et 0,5, et en particulier lorsque ce rapport est entre 0,05 et 2.

Les inventeurs ont par ailleurs mis en évidence que les dispersions de l'invention présentent, outre leur stabilité, une propriété spécifique inattendue. Plus précisément, il s'avère que, compte tenu de l'utilisation spécifique des polymères P, si on élimine le milieu dispersant d'une dispersion selon l'invention, notamment par évaporation, on obtient un mélange essentiellement constitué des particules et des polymères qui présente la capacité étonnante de pouvoir être redispersée dans un milieu dispersant pour former à nouveau une dispersion stabilisée de type organosol (sous réserve toutefois que le nouveau milieu dispersant soit compatibles avec les blocs B des polymères P utilisés). Cette propriété des organosols de l'invention est tout à fait surprenante au vu des résultats obtenus avec les agents de stabilisation de type tensioactifs (acide oléique ou acide isostéarique notamment) avec lesquels une évaporation du milieu dispersion conduit, au contraire, à une agrégation irréversible des particules.

Les compositions du type obtenues par élimination du milieu organique présent dans un organosol selon l'invention, qui comprennent des particules minérales et des polymères P du type précité, et qui sont dispersibles en milieu organique pour former une nouvelle dispersion de particules minérales stabilisée, constituent un autre objet de la présente invention. Ces compositions redispersibles se présentent le plus souvent sous la forme de compositions huileuses. Le plus souvent, ces compositions sont essentiellement constituées par un mélange de particules organique et de polymères amphiphiles séquencés.

Le procédé de préparation des compositions redispersibles précitées, par élimination du milieu organique présent dans une dispersion de particules minérales selon l'invention, notamment par évaporation de ce milieu organique, constitue un autre objet particulier de,la présente invention.

Pour la préparation de dispersions redispersibles du type précité à base de particules et de polymères P, on peut avantageusement former un organosol selon l'étape (A) précitée, en utilisant spécifiquement à titre de milieu dispersant un solvant organique hydrophobe de faible point d'ébullition, tel que par exemple le toluène. La mise en oeuvre d'un tel milieu dispersant volatil permet facilite son élimination ultérieure, qui peut être réalisée par simple évaporation du solvant volatil pour fournir directement la compositions redispersible recherchée.

Quel que soit leur mode d'obtention, les compositions redispersibles de l'invention, peuvent être utilisées pour la préparation de dispersions de particules dans tout milieu organique, y compris dans des milieux organiques non compatibles avec la mise en oeuvre de l'étape (A), tels que des milieux organiques hydrophiles.

Dans ce cadre, l'invention fournit ainsi, selon encore un autre aspect, un procédé de préparation d'une dispersion de particules minérales du type, dans laquelle le milieu organique peut indifféremment être hydrophobe ou hydrophile, ce procédé comprenant une étape (B) de dispersion d'une composition redispersible du type précité au sein dudit milieu organique, hydrophobe ou hydrophile.

Selon un mode de réalisation spécifique, l'invention fournit ainsi en particulier un procédé de préparation d'une dispersion de particules minérales dans un milieu dispersant organique hydrophile, ce procédé comprenant :
- la mise en oeuvre de l'étape (A) précitée avec un premier milieu dispersant ayant de préférence un faible point d'ébullition, puis
- l'élimination dudit premier milieu dispersant (par exemple par évaporation) ce par quoi on obtient un composition redispersible du type précité ; puis
- la mise en oeuvre de l'étape (B) précitée en utilisant ledit milieu dispersant organique hydrophile à titre de milieu dispersant.

Quel que soit leur mode de préparation, les organosols de l'invention trouvent des applications dans un grand nombre de domaines techniques.

En particulier, ces organosols se révèlent particulièrement bien adaptées pour la préparation de compositions solvantées, telles que des peintures notamment.

On peut également utiliser certains des organosols de l'invention pour la formulation de compositions dans le domaine de la cosmétique, sous réserve d'employer des particules cosmétiquement acceptables. Dans ce type d'application, le milieu organique dispersant de l'organosol est de préférence de type huile végétale, huile de silicone et/ou huile minérale.

Selon un mode de réalisation particulier, les organosols de l'invention peuvent aussi être utilisés pour effectuer des réactions catalysées en milieu solvant (dans ce cas, les particules utilisées sont des particules présentant des propriétés catalytiques, en général des particules présentant en surface des espèces catalytiques actives, déposées sur ces particules ou faisant partie de la structure de la particule)

Ces différentes utilisations constituent, selon un autre aspect, un autre objet particulier de la présente invention.

Différents avantages et caractéristiques intéressantes de l'invention ressortiront encore d'avantage des exemples illustratifs exposés ci-après.

### EXEMPLES

### A- Préparations de polymères séquencés amphiphile selon l'invention.

### Exemple A1 :

### Synthèse d'un copolymère séquencé de type PEHA-PAA (masse moléculaire théorique du bloc poly(acrylate de 2-ethylhexyle) : 2250 masse moléculaire théorique du bloc poly(acide acrylique): 125)

### • étape 1 : synthèse d'un bloc PEHA à extrémité réactive (xanthate)

Dans un réacteur en verre de 2 litres maintenu sous argon, muni d'une agitation de type impeller et d'un réfrigérant, on a introduit 237,8 g d'éthanol, 37,03 g d' *O-*ethyl-S-(1-methoxycarbonyl)ethyl)xanthate (CH₃CHCO₂CH₃)S(C=S)OEt, et 400 g d'acrylate de 2-ethylhexyle.

Le milieu obtenu a été porté à 75°C, et 4,52 g d'azo-bis(methylisobutyronitrile) (AMBN) ont été ajoutés au mélange réactionnel, puis on a laissé la réaction se poursuivre pendant 9 heures à 75°C.

Des analyses par RMN¹H et par chromatographie en phase gazeuse indiquent que le monomère acrylate a été totalement polymérisé. La masse molaire du polymère obtenu, telle que mesurée par chromatographie d'exclusion stérique (étalonnage par du poly(styrène)) est de 2350 g/mol.

### • étape 2 : synthèse du polymère dibloc PAA-PEHA

Au milieu obtenu à l'issue de la première étape, maintenu à 75°C, on a ajouté 0,11 g d'AMBN, dilués dans 1g d'éthanol. Immédiatement après, on a introduit dans le milieu, en une fois, une solution de 22,2 g d'acide acrylique dans 15 g d'éthanol. On a laissé le milieu ainsi obtenu réagir pendant 2 heures à 75°C, puis on a ajouté 0,11 g d'AMBN dilués dans 1g d'éthanol. Suite à ce nouvel ajout d'AMBN, le milieu réactionnel a été maintenu à 75°C pendant 8 heures.

A l'issue de cette réaction, on a obtenu un polymère dibloc PAA-PEHA. Une analyse RMN ¹H indique que la composition du copolymère final correspond à celle attendue. Une analyse HLPC indique la consommation totale de l'acide acrylique introduit.

### Exemple A2

### Synthèse d'un copolymère séquencé de type PAA-PEHA (masse moléculaire théorique du bloc poly(acrylate de 2-ethylhexyle) : 2250 masse moléculaire théorique du bloc poly(acide acrylique): 125)

### • étape 1 : synthèse d'un bloc PAA à extrémité réactive (xanthate)

Dans un réacteur en verre de 2 litres maintenu sous argon, muni d'une agitation de type impeller et d'un réfrigérant, on a introduit 28,8 g d'éthanol, 33,32 g de *O-*ethyl-*S*-(1-methoxycarbonyl)ethyl) xanthate (CH₃CHCO₂CH₃)S(C=S)OEt et 20 g d'acide acrylique. La solution a été portée à 75°C, et 0,3 g d'azo-bis(methylisobutyronitrile) (AMBN) ont été ajoutés au mélange réactionnel. Le milieu réactionnel obtenu a alors été maintenue pendant 9 heures à 75°C

Des analyses par RMN¹H et par chromatographie en phase gazeuse indiquent que le monomère a été totalement polymérisé. La masse molaire du polymère obtenu, telle que mesurée par chromatographie d'exclusion stérique (étalonnage par du poly(oxyde d'éthylène)) est de 400 g/mol.

### • étape 2 : synthèse du polymère dibloc PAA-PEHA

Au milieu issu de la première étape, maintenu à 75°C, on a ajouté 1,8 g d'AMBN dilués dans 1g d'éthanol. Immédiatement après, on a introduit dans le milieu, en une seule fois, une solution de 360 g d'acrylate de 2-ethylhexyle dans 242,4 g d'éthanol, puis on a laissé réagir pendant 2 heures après l'introduction de la solution d'acrylate.

On a ensuite introduit 1,8 g d'AMBN dilués dans 1g d'éthanol. Suite à ce nouvel ajout d'AMBN, le milieu réactionnel a été maintenu à 75°C pendant huit heures.

A l'issue de cette réaction, on a obtenu le polymère dibloc PAA-PEHA recherché. Une analyse RMN ¹H indique que la composition du copolymère final correspond à celle attendue. Une analyse par chromatographie en phase gazeuse indique la consommation totale de l'acrylate de 2-ethylhexyle.

### Exemple A3 :

### Synthèse d'un copolymère séquencé de type PAA-PEHA (masse moléculaire théorique du bloc poly(acrylate de 2-ethylhexyle): 1125 masse moléculaire théorique du bloc poly(acide acrylique) : 250)

### • étape 1 : synthèse d'un bloc PAA à extrémité réactive (xanthate)

Dans un réacteur en verre de 2 litres maintenu sous argon, muni d'une agitation de type impeller et d'un réfrigérant, on a introduit 20,4 g d'éthanol, 16,66 g de *O-*ethyl-*S*-(1-methoxycarbonyl)ethyl) xanthate (CH₃CHCO₂CH₃)S(C=S)OEt et 20 g d'acide acrylique.

Le milieu obtenu a été porté à 75°C, et 1,17 g d' azo-bis(methylisobutyronitrile) (AMBN) ont été ajoutés au mélange réactionnel, puis on a laissé la réaction se poursuivre pendant neuf heures à 75°C.

Des analyses par RMN¹H et par chromatographie en phase gazeuse indiquent que le monomère acide acrylique a été totalement polymérisé. La masse molaire du polymère obtenu, telle que mesurée par chromatographie d'exclusion stérique est de 550 g/mol.

### • étape 2 : synthèse du polymère dibloc PAA-PEHA

Au milieu obtenu à l'issue de la première étape, maintenu à 75°C, on a ajouté 0,45 g d'AMBN, dilués dans 1g d'éthanol. Immédiatement après, on a introduit dans le milieu, en une fois, une solution de 60 g d'acrylate de 2-éthylhexyle dans 60,6 g d'éthanol. On a laissé le milieu ainsi obtenu réagir pendant 2 heures à 75°C, puis on a ajoutés 0,45 g d'AMBN dilués dans 1g d'éthanol. Suite à ce nouvel ajout d'AMBN, le milieu réactionnel a été maintenu à 75°C pendant 8 heures.

A l'issue de cette réaction, on a obtenu un polymère dibloc PAA-PEHA. Une analyse RMN ¹H indique que la composition du copolymère final correspond à celle attendue. Une analyse par chromatographie en phase gazeuse indique la consommation totale de l'acrylate de 2-ethylhexyle.

### Exemple A4:

### Synthèse d'un copolymère séquencé de type PEHA-PAA (masse moléculaire théorique du bloc poly(acide acrylique) : 250 masse moléculaire théorique du bloc poly(acrylate de 2-ethylhexyle): 4500)

### • étape 1 : synthèse d'un bloc PEHA à extrémité réactive (xanthate)

Dans un réacteur en verre de 2 litres maintenu sous argon, muni d'une agitation de type impeller et d'un réfrigérant, on a introduit 227,7 g d'éthanol, 18,51 g de *O-*ethyl-*S*-(1-methoxycarbonyl)ethyl) xanthate (CH₃CHCO₂CH₃)S(C=S)OEt et 400 g d'acrylate de 2-ethylhexyle.

Le milieu obtenu a été porté à 75°C, et 4,52 g d' azo-bis(methylisobutyronitrile) (AMBN) ont été ajoutés à ce mélange réactionnel, puis on a laissé la réaction se poursuivre pendant neuf heures à 75°C.

Des analyses par RMN¹H et par chromatographie en phase gazeuse indiquent que le monomère acrylate a été totalement polymérisé. La masse molaire du polymère obtenu, telle que mesurée par chromatographie d'exclusion stérique est de 4300 g/mol.

### • étape 2 : synthèse du polymère dibloc PAA-PEHA

Au milieu obtenu à l'issue de la première étape, maintenu à 75°C, on a ajouté 0,11 g d'AMBN dilués dans 1g d'éthanol. on a introduit dans le milieu, en une fois, une solution de 22,2 g d'acide acrylique dans 15 g d'éthanol. On a laissé le milieu ainsi obtenu réagir pendant 2 heures à 75°C, puis on a ajoutés 0,11 g d'AMBN dilués dans 1g d'éthanol. Suite à ce nouvel ajout d'AMBN, le milieu réactionnel a été maintenu à 75°C pendant 8 heures.

A l'issue de cette réaction, on a obtenu un polymère dibloc PAA-PEHA. Une analyse RMN ¹H indique que la composition du copolymère final correspond à celle attendue. Une analyse par chromatographie en phase gazeuse indique la consommation totale de l'acide acrylique.

### Exemple A5 :

### Synthèse d'un copolymère séquencé de type PAA-PEHA (masse moléculaire théorique du bloc poly(acrylate de 2-ethylhexyle) : 1125 masse moléculaire théorique du bloc poly(acide acrylique): 250)

### • étape 1 : synthèse d'un bloc PEHA à extrémité réactive (xanthate)

Dans un réacteur en verre de 2 litres maintenu sous argon, muni d'une agitation de type impeller et d'un réfrigérant, on a introduit 135,5 g d'éthanol, 38,9 g de *O-*ethyl-S-(1-methoxycarbonyl)ethyl) xanthate (CH₃CHCO₂CH₃)S(C=S)OEt et 210 g d'acrylate de 2-ethylhexyle.

Le milieu obtenu a été porté à 75°C, et 2,75 g d'azo- bis(methylisobutyronitrile) (AMBN) sont ajoutés au mélange réactionnel, puis on a laissé la réaction se poursuivre pendant 9 heures à 75°C.

Des analyses par RMN¹H et par chromatographie en phase gazeuse indiquent que le monomère a été totalement polymérisé. La masse molaire du polymère obtenu, telle que mesurée par chromatographie d'exclusion stérique est de 1300 g/mol.

### • étape 2 : synthèse du polymère dibloc PAA-PEHA

Au milieu obtenu à l'issue de la première étape, maintenu à 75°C, on a ajouté 0,23 g d'AMBN dilués dans 1g d'éthanol. Immédiatement après, on a introduit dans le milieu, en une fois, une solution de 46,67 g d'acide acrylique dans 31,4 g d'éthanol. On a laissé le milieu ainsi obtenu réagir pendant 2 heures à 75°C, puis on a ajoutés 0,23 g d'AMBN dilués dans 1g d'éthanol. Suite à ce nouvel ajout d'AMBN, le milieu réactionnel a été maintenu à 75°C pendant 8 heures.

A l'issue de cette réaction, on a obtenu un polymère dibloc PAA-PEHA. Une analyse RMN ¹H indique que la composition du copolymère final correspond à celle attendue. Une analyse HLPC indique la consommation totale de l'acide acrylique introduit.

### Exemple A6 :

### Synthèse d'un copolymère dibloc de type PIO-PAA (poly(acrylate d'isooctyle) - poly(acide acrylique)) (masse moléculaire théorique du bloc poly(acrylate d'isooctyle)- : 2250 masse moléculaire théorique du bloc poly(acide acrylique): 125))

### • étape 1 : synthèse d'un bloc PAI à extrémité réactive (xanthate)

Dans un ballon en verre bicol de 100 ml maintenu sous argon, muni d'une agitation magnétique et d'un réfrigérant, on a introduit 18,7 g d'éthanol, 4,7 g de tetrahydrofurane, 3,34 g de *O*-ethyl-*S*-(1-methoxycarbonyl)ethyl) xanthate (CH₃CHCO₂CH₃)S(C=S)OEt et 36 g d'acrylate d'isooctyle.

Le milieu ainsi obtenu a été porté à une température à 75°C, et 3,3 g d' azo-bis(methylisobutyronitrile) (AMBN) sont ajoutés au mélange réactionnel, puis le milieu réactionnel a été maintenue pendant neuf heures à 75°C.

Des analyses par RMN¹H et par chromatographie en phase gazeuse indiquent que le monomère a été totalement polymérisé. La masse molaire du polymère obtenu, telle que mesurée par chromatographie d'exclusion stérique est de 2400 g/mol.

### • étape 2 : synthèse du polymère dibloc PAI-PAA

Au milieu obtenu à l'issue de la première étape, maintenu à 75°C, on a ajouté 0,8 g d'AMBN dilués dans 1g d'éthanol. Immédiatement après, on a introduit dans le milieu, en une fois, une solution de 4 g d'acide acrylique et de 3,3 g d'éthanol. On a ensuite laissé le milieu réagir pendant 3 heures après puis on a ajoutés 0,8 g d'AMBN dilués dans 1g d'éthanol. Suite à ce nouvel ajout d'AMBN, le milieu réactionnel a été maintenu à 75°C pendant six heures.

A l'issue de cette réaction, on a obtenu un polymère dibloc PAI-PAA. Une analyse RMN ¹H indique que la composition du copolymère final correspond à celle attendue. Une analyse par chromatographie HPLC indique la consommation totale de l'acide acrylique.

### Exemple A7 :

### Synthèse d'un copolymère dibloc de type PAB-PAA (poly(acrylate de butyle) - poly(acide acrylique)) (masse moléculaire théorique du bloc poly(acrylate de butyle)-: 2250 masse moléculaire théorique du bloc poly(acide acrylique): 125)

### • étape 1 : synthèse d'un bloc PAB à extrémité réactive (xanthate)

Dans un ballon en verre bicol de 500 ml maintenu sous argon, muni d'une agitation magnétique et d'un réfrigérant, on a introduit 83 g d'éthanol, 12,94 g de *O*-ethyl-S-(1-methoxycarbonyl)ethyl) xanthate (CH₃CHCO₂CH₃)S(C=S)OEt, et 140 g d'acrylate de butyle.

Le milieu ainsi obtenu a été porté à 75°C, et 1,5 g d'azo-bis(methylisobutyronitrile) (AMBN) ont été ajoutés au mélange réactionnel, puis le milieu obtenu a été maintenu pendant 9 heures à 75°C.

Des analyses par RMN¹H et par chromatographie en phase gazeuse indiquent que le monomère a été totalement polymérisé. La masse molaire du polymère obtenu, telle que mesurée par chromatographie d'exclusion stérique est de 2150 g/mol.

### • étape 2 : synthèse du polymère dibloc PAB-PAA

On a placé une solution de 36 g de polymère issu de la première étape dans 29,4 g d'éthanol dans un réacteur en verre de 100 ml maintenu à 75°C, puis on a introduit dans le milieu 5 mg d'AMBN. Immédiatement après, on a introduit au milieu réactionnel, en une seule fois, une solution de 2 g d'acide acrylique dans 1,95 g d'éthanol.

On a laissé le milieu obtenu réagir pendant 3 heures après l'introduction de la solution d'acide acrylique, puis on a ajouté 5 mg d'AMBN. Suite à ce nouvel ajout d'AMBN., le milieu réactionnel a été maintenu à 75°C pendant six heures.

A l'issue de cette réaction, on a obtenu un polymère dibloc PAB-PAA. Une analyse RMN ¹H indique que la composition du copolymère final correspond à celle attendue. Une analyse par chromatographie HPLC indique la consommation totale de l'acide acrylique.

### Exemple A8 :

### Synthèse d'un copolymère dibloc de type copolymère statistique (acrylate de 2-ethylhexyle/acrylate de 2-hydroxyéthyle)-poly(acide acrylique) (masse moléculaire théorique du bloc copolymère statistique: 2250 masse moléculaire théorique du bloc poly(acide acrylique): 125)

### • étape 1: synthèse d'un bloc copolymère statistique (acrylate de 2-ethylhexylelacrylate de 2-hydroxyéthyle) à extrémité réactive (xanthate)

Dans un ballon en verre de 100 ml maintenu sous argon, muni d'une agitation magnétique et d'un réfrigérant, on a introduit 38,7 g d'éthanol, 6,26 g de O-ethyl-*S*-(1-methoxycarbonyl)ethyl) xanthate (CH₃CHCO₂CH₃)S(C=S)OEt, 47,8 g d'acrylate de 2-ethylhexyle et 17,16 g d'acrylate de 2-hydroxyéthyle.

Le milieu obtenu a été porté à 75°C, 0,7 g d'azo-bis(methylisobutyronitrile) (AMBN) ont été ajoutés au mélange réactionnel, puis on a laissé le milieu réagir pendant neuf heures à 75°C.

Des analyses par RMN¹H et par chromatographie en phase gazeuse indiquent que les monomères introduits ont totalement polymérisé. La masse molaire du polymère obtenu, telle que mesurée par chromatographie d'exclusion stérique est de 2350 g/mol (avec un rapport M_{w}/Mₙ de 1,60).

### • étape 2 : synthèse du polymère dibloc

Au milieu issu de la première étape, maintenu à 75°C, on a ajouté 0,015 g d'AMBN dilués dans 1g d'éthanol. Immédiatement après, un mélange contenant 3 g d'acide acrylique et 2,92 g d'éthanol a été additionné en une fois dans le milieu.

On a ensuite laissé le milieu réactionnel réagir pendant 3 heures après l'introduction de la solution d'acide acrylique, puis on a ajouté 0,015 g d'AMBN dilués dans 1g d'éthanol. Suite à ce nouvel ajout d'AMBN, la réaction a été maintenue à 75°C pendant huit heures.

A l'issue de cette réaction, on a obtenu le polymère dibloc recherché.

### Exemple A9 :

### Synthèse d'un copolymère séquencé de type PAA-PEHA (masse moléculaire théorique du bloc poly(acrylate de 2-ethylhexyle) : 1125 masse moléculaire théorique du bloc poly(acide acrylique): 125)

### • étape 1 : synthèse d'un bloc PEHA à extrémité réactive (xanthate)

Dans un réacteur en verre de 2 litres maintenu sous argon, muni d'une agitation de type impeller et d'un réfrigérant, on a introduit 135,5 g d'éthanol, 38,9 g de *O-*ethyl-*S*-(1-methoxycarbonyl)ethyl) xanthate (CH₃CHCO₂CH₃)S(C=S)OEt et 210 g d'acrylate de 2-ethylhexyle.

Le milieu obtenu a été porté à 75°C, et 2,50 g d'azo- bis(methylisobutyronitrile) (AMBN) sont ajoutés au mélange réactionnel. Le milieu réactionnel a alors été maintenu pendant 9 heures à 75°C.

Des analyses par RMN¹H et par chromatographie en phase gazeuse indiquent que le monomère a été totalement polymérisé. La masse molaire du polymère obtenu, telle que mesurée par chromatographie d'exclusion stérique est de 1200 g/mol.

### • étape 2 : synthèse du polymère dibloc PEHA-PAA

Au polymère issu de la première étape, maintenu à 75°C, sont ajoutés 0,12 g d'AMBN dilués dans 1g d'éthanol. Immédiatement après, un mélange contenant 23,33 g d'acide acrylique et 15,71 g d'éthanol est additionné en une fois. 2 heures après l'introduction de la solution d'acide acrylique, sont ajoutés 0,12 g d'AMBN dilués dans 1g d'éthanol. Suite à ce nouvel ajout, la réaction est maintenue à 75°C pendant huit heures.

Une analyse RMN ¹H indique que la composition du copolymère final correspond à celle attendue. Une analyse par chromatographie HPLC indique la consommation totale de l'acide acrylique.

### B- Préparation d'organosols selon l'invention utilisant les polymères précédemment synthétisés

### Exemple B1

### Organosol de particules de CeO2 dans l'acétate de butyle utilisant le polymère séquencé PAA-PEHA de l'exemple A1 (Organosol O1)

### • Préparation de l'organosol O1

Dans un réacteur de 2L muni d'un réfrigérant et d'une agitation mécanique de type ancre, on a introduit, sous agitation, 200 ml d'un sol aqueux de CeO2 à 20 g/litre, ayant un diamètre hydrodynamique de 5 nm, puis une solution de 33,47 g du copolymère séquencé PEHA-PAA de l'exemple A1, et enfin 200ml d'acétate de butyle.

Les quantités relatives de polymère et de CeO2 utilisées dans le milieu ainsi réalisé correspondent à un rapport molaire (COOH porté par les polymères/CeO2 total) de 1.

Le mélange réalisé a été laissé sous agitation à 60°C pendant 6 heures, de façon à laisser se réaliser le transfert des particules du sol aqueux vers la phase organique.

Après arrêt de l'agitation et refroidissement du milieu jusqu'à la température ambiante (25°C environ), on a laissé le milieu obtenu décanter pendant une nuit (de l'ordre de 12 heures).:

Après décantation, la phase aqueuse a été séparée de la phase organique, en soutirant cette phase aqueuse (plus dense que la phase organique) par la vanne de fond du réacteur. On a ainsi obtenu un organosol O1 (phase organique séparée).

Cet organosol O1 présente une perte au feu de 10,6 % (calculée sur l'organosol totalement séché), ce qui correspond à un taux de transfert des particules du sol aqueux initial vers le milieu organique de l'ordre égal à 94,8%).

### • Stabilité de l'organosol O1

L'organosol obtenu présente une bonne stabilité. Ainsi, après un stockage de cet organosol de 6 mois à température ambiante, on ne constate pas visuellement de sédimentation des particules.

### • Possibilité de "séchage" de l'organosol O1 et de redispersion de la composition séchée obtenue en milieu organigue

Afin d'illustrer les propriétés spécifiques des organosols de l'invention, à savoir la possibilité d'éliminer leur milieu dispersant sans affecter la dispersion des particule minérales, on a effectué le séchage de l'organosol O1 obtenu précédemment.

Dans ce cadre l'organosol a été totalement séché par évaporation sous vide de l'acétate de butyle, jusqu'à obtention d'une masse constante. On a ainsi obtenu une huile jaune pâle, transparente et visqueuse, composée essentiellement de particules de CeO₂ et du copolymère de l'exemple A1.

La structure de l'huile ainsi obtenue a été analysée par diffusion de rayons X aux petits angles. Le spectre obtenu fait apparaître un pic d'ordre très intense à une valeur de vecteur d'onde q de 170 Å, caractéristique d'une bonne dispersion des objets minéraux dans le produit. Ces résultats indiquent que la composition obtenue est un milieu concentré et ordonné où les particules sont régulièrement espacées au sein du polymère. Schématiquement, les particules sont présentes dans cette composition à l'état de particules sensiblement individualisées, et ce malgré la concentration du milieu, ce qui semble pouvoir être attribué à un ancrage des polymères sur les particules par l'intermédiaire des fonctions acide acrylique portées par les polymères.

A titre de comparaison, on a effectué un séchage du sol aqueux initial, en l'absence de polymère séquencé, et on a soumis la poudre séchée obtenue à la même analyse de diffusion de rayons X aux petits angles, qui fournit un spectre radicalement différent. Ainsi, au lieu d'un pic d'ordre bien défini, on observe un plateau avec un maximum peu prononcé correspondant à une distance caractéristique de l'ordre du diamètre moyen des particules (autour de 74 Å), avec une remontée de la courbe aux petits angles, qui traduit des phénomènes d'agglomération, non observés dans le cas de l'organosol séché

Par ailleurs, l'organosol séché obtenu après élimination de l'acétate de butyle a été à nouveau dispersé dans de l'acétate de butyle à deux concentrations différentes (0.5% et 1,6% en masse). Les diamètres hydrodynamiques moyens des objets dispersés dans cet organosols ont été mesurés par diffusion quasi-élastique de la lumière, les résultats obtenus sont indiqués dans le Tableau Il ci-dessous.

**Tableau 2**

| **Sols obtenus par redispersion de l'organosol O1 après séchage** | |
|---|---|
| Concentration massique | Diamètre hydrodynamique des objets |
| de CeO₂ | dispersés dans l'organosol obtenu |
| 0.5% | Entre 12 et 18nm. |
| 1.6% | Entre 15 et 25 nm. |

Les diamètres hydrodynamiques mesurés indiquent une bonne redispersion du CeO₂ dans le solvant, malgré l'étape de séchage total à laquelle le sol a été soumise. A titre indicatif, le diamètre hydrodynamique moyen des objets dispersés dans le sol O1 avant séchage est de l'ordre de 15 nm.

### Exemple B2

### Organosol de particules de CeO2 dans l'acétate de butyle utilisant le polymère séquencé PAA-PEHA de l'exemple A2 (Organosol O2)

Dans un réacteur de 2L muni d'un réfrigérant et d'une agitation mécanique de type ancre, on a introduit 150 ml d'un sol aqueux de CeO₂ à 20 g/litre tel qu'utilisé dans l'exemple B1, puis on a introduit dans le réacteur sous agitation une solution de 9,55 g du copolymère séquencé PAA-PEHA de l'exemple A2 dans 150 ml d'acétate de butyle.

Les quantités relatives de polymère et de CeO₂ utilisées dans le milieu ainsi réalisé correspondent à un rapport molaire (COOH porté par les polymères/CeO2 total) de 0,4.

Le mélange réalisé a été agité à 60°C pendant 6 heures. Après arrêt de l'agitation et diminution de la température jusqu'à l'ambiante (25°C environ), on a laissé le mélange décanter pendant une nuit (de l'ordre de 12 heures). Après décantation, la phase aqueuse a été séparée de la phase organique par soutirement de la phase aqueuse par la vanne de fond du réacteur, comme dans l'exemple B1.

On a ainsi obtenu un organosol O2 (phase organique séparée), qui présente, à l'état totalement séché, une perte au feu de 22,9%, ce qui correspond à un taux de transfert égal à 98,6%.

Comme dans l'exemple 1, l'organosol obtenu présente une bonne stabilité sans conduire à des phénomènes de sédimentation au stockage.

### Exemple B3

### Organosol de particules de CeO₂ dans l'Isopar L, utilisant le polymère séquencé PAA-PEHA de l'exemple A2(Organosol 03)

On a répété l'exemple B2, à la différence près que les 150 mL d'acétate de butyle utilisés ont été remplacés par 150 ml d'Isopar L.

On a ainsi obtenu un Organosol 03 de particules deCeO₂ dans l'Isopar L, avec un taux de transfert de 96,1 %.

Là encore, l'organosol obtenu présente une bonne stabilité au stockage.

### Exemple B4

### Organosol de particules de CeO2 dans l'Exxsol D40, utilisant le polymère séquencé PAA-PEHA de l'exemple A2(Organosol O4)

On a répété le protocole des exemples B2 et B3, en utilisant cette fois 150 ml d'Exxsol D40.

On a ainsi obtenu un Organosol 04 de particules deCeO2 dans l'Exxsol D40, avec un taux de transfert égal à 97,4 %.

Les résultats des exemples B2, B3 et B4 exposés ci-dessus illustrent bien l'aptitude que présentent les polymères séquencés de type PAA-PEHA à réaliser des organosols à base de particules de CeO2 dans de nombreux types de solvants de polarités différentes.

### Exemple B5

### Organosol de particules de CeO2 dans l'acétate de butyle, utilisant le polymère séquencé de l'exemple A8 (Organosol 05)

Dans un réacteur de 2L muni d'un réfrigérant et d'une agitation mécanique de type ancre, on a introduit, sous agitation, 150 ml d'un sol aqueux de CeO2 à 20 g/litre tel qu'utilisé dans l'exemple B1, puis une solution de 9,55 g de copolymère de l'exemple A8 dans 150 ml d'acétate de butyle.

Les quantités relatives de polymère et de CeO₂ utilisées dans le milieu ainsi réalisé correspondent à un rapport molaire (COOH porté par les polymères/CeO2 total) de 0,4.

Le mélange est été laissé sous agitation à 60°C pendant 6 heures. Après arrêt de l'agitation et diminution de la température jusqu'à l'ambiante (25°C environ), le mélange est laissé à décanter pendant une nuit (de l'ordre de 12 heures). Après décantation, la phase aqueuse a été séparée de la phase organique par soutirement de la phase aqueuse par la vanne de fond du réacteur.

Une perte au feu mesurée sur l'organosol totalement séché permet d'estimer un taux de transfert égal à 94,1%.

Comme dans les exemples précédents l'organosol obtenu est stable au stockage.

### EXEMPLES COMPARATIFS

### EXEMPLE COMPARATIF 1

### comparaison de la stabilité des organosols obtenus avec des copolymères séquencés selon l'invention et avec des agents tensioactifs usuels

Pour mettre en évidence l'amélioration significative de la stabilité des organosols que procurent les copolymères de l'invention par rapport aux agents tensioactifs usuels, on a comparé différents organosols utilisant ces deux types d'agents de stabilisation.

Plus précisément, on a préparé plusieurs organosol de particules de CeO₂ dans de l'Exxsol D40, en utilisant à titre d'agent stabilisant un mélange M comprenant de copolymère séquencé de l'exemple A1 et/ou de l'acide isostéarique, avec des proportions variables en copolymère et en acide isostéarique, reportées dans le tableau III ci-après. Dans un cas particulier (témoin), le mélange M utilisé contient 100% d'acide stéarique, sans polymère. Dans les autres cas, le mélange M contient un polymère selon l'invention en une proportion allant de 10 à 100% en mole.

Dans chacun des cas, l'organosol a été réalisé selon le protocole ci-après.

Dans un réacteur de 75 ml muni d'un réfrigérant et d'une agitation magnétique, on introduit 18 ml de sol aqueux de CeO2 à 5,77 g/litre. Une solution de 18 ml d'Exxsol D40 contenant le mélange M a ensuite été introduite dans le réacteur. Dans chacun des cas, la masse de polymère (m_{P}) et la masse d'acide isostéarique (m_{AIS}) introduites ont été adaptées de façon à maintenir dans le milieu un rapport molaire (COOH porté par les polymères/CeO2 total) fixe, égal à 0,8. Les masses m_{P} et M_{AIS} correspondantes sont reportées dans le Tableau III ci-après.

Le mélange obtenu a été agité à 60°C pendant 1 heure. Après arrêt de l'agitation et diminution de la température jusqu'à l'ambiante (25°C), le mélange- est laissé à décanter pendant 12 heures. Après décantation, la phase aqueuse a été séparée de la phase organique par prélèvement de la phase organique à la pipette.

Chacune des phases organiques ainsi prélevées constitue un organosol dont la stabilité a été testée en cyclage chaud/froid (-20 C; +90°C).

A cet effet, les différents échantillons d'organosols prélevés ontt été placés dans une étuve et soumis à une série de cycles de température, chacun de ces cycles étant définis comme suit :
- maintien en température à 90°C pendant 1 heure
- descente en température de 90°C jusqu'à -20°C en une heure ;
- remontée immédiate en température de -20°C jusqu'à + 90°C en une heure (avant reprise du cycle suivant: nouveau plateau d'une heure- descente à - 20°C et remontée à + 90°C).

Pour chaque échantillon, on a noté le temps au bout duquel les sols organiques deviennent troubles et où on observe la formation d'un dépôt blanc, caractéristique de la perte de la stabilité. La durée de la stabilité précédant l'apparition du trouble et du dépôt blanc qui a été observée dans chaque cas est reportée dans le tableau III ci -dessous.

**Tableau III: essais de stabilité comparés de différents organosols de particules de CeO2 dans de l'Exxsol D40**

| Teneur en polymère | Masse de polymère | Masse d'acide | Durée de la |
|---|---|---|---|
| dans le mélange M | (m_{P}) | isostéarique (m_{AIS}) | stabilité |
| Pas de polymère (témoin) | - | 0,137 g | 48 h |
| 10 % en mole | 0,066 g | 0,123 g | 72 h |
| 25 % en mole | 0,165 g | 0,103 g | 96 h |
| 50 % en mole | 0,329 g | 0,068 g | 100 h |
| 75 % en mole | 0,494 g | 0,034 g | 216 h |
| 100 % en mole | 0,659 g | - | 240 h |

Cet exemple met ainsi en évidence que la durée de stabilité double lorsqu'on remplace 25% en mole de l'acide isostéarique du témoin par le polymère de l'exemple A1. Elle est multipliée par 5 lorsqu'on remplace 100% de l'acide isostéarique par le polymère.

### EXEMPLE COMPARATIF 2

### Comparaison des propriétés des copolymères séquencés selon l'invention et de copolymères statistiques de même composition

### • Préparation d'un copolymère statistique P_{STAT} à base d'unités acrylate de 2-ethylhexyle et acide acrylique ayant globalement la même composition que le copolymère séquencé des exemple A1 et A2

A des fins de comparaison, un polymère statistique à base d'unités acide acrylique et acrylate de 2-ethylhexyle a été synthétisé dans les conditions suivantes.

Dans un réacteur en verre de 500 ml maintenu sous argon, muni d'une agitation de type impeller et d'un réfrigérant, on introduit 99,4 g d'éthanol, 18,51 g de O-ethyl-*S*-(1-methoxycarbonyl)ethyl) xanthate (CH₃CHCO₂CH₃)S(C=S)OEt, 200 g d'acrylate de 2-ethylhexyle et 11,11 g d'acide acrylique.

La solution est mise en température à 75°C, et 2,26 g d' azo-bis(methylisobutyronitrile) (AMBN) sont ajoutés au mélange réactionnel. Après trois heures de réaction, 0,056 g d'AMBN sont rajoutés à la réaction. La réaction est maintenue encore pendant cinq heures à cette température après ce dernier ajout d'AMBN.

En fin de réaction, une analyse par RMN¹N indique une conversion totale des monomères. La masse molaire en nombre Mn du polymère obtenu, telle que mesurée par chromatographie d'exclusion stérique dans le THF (étalonnage par le polystyrène) est de 2300 g/mol.

### • Essais de transfert des particules de Ce02 d'un sol aqueux vers de l'acétate de butyle en utilisant le polymère statistique P_{STAT}

Dans un réacteur de 500ml muni d'un réfrigérant et d'une agitation mécanique de type ancre, on a introduit 100 ml d'un sol aqueux de CeO2 à 20 g/litre tel qu'utilisé dans l'exemple B1, puis une solution de 6,47 g de copolymère A9 dans 100 ml d'acétate de butyle est ensuite introduite dans le réacteur.

Les quantités relatives de polymère et de CeO₂ utilisées dans le milieu ainsi réalisé correspondent à un rapport molaire (COOH porté par les polymères/CeO2 total) de 0,4.

Le mélange a été mis sous agitation à 60°C pendant 6 heures. Après arrêt de l'agitation et diminution de la température jusqu'à l'ambiante, le mélange a été laissé à décanter pendant une nuit. Après décantation, la phase aqueuse a été séparée de la phase organique par soutirement de la phase aqueuse par la vanne de fond du réacteur.

La perte au feu mesurée sur l'organosol totalement séché permet d'établir que le taux de transfert est égal à 11,1%.

Le même protocole a été réalisé, mais en utilisant cette fois 12,73 g de polymère au lieu de 6,47 g, pour obtenir un rapport molaire (COOH porté par les polymères/CeO2 total) de 0,8. On a ainsi obtenu un rendement du transfert (déterminé à partir de la mesure de la perte au feu de l'organosol totalement séché) de 25,7 %.

Les taux de transferts très faibles obtenus avec l'emploi du polymère P_{STAT} montrent clairement que ce polymère statistique est un agent de transfert de phase médiocre, qui conduit à des rendements de transfert beaucoup plus faibles que dans le cas de l'utilisation du polymères des exemple A1 et A2 (voir les exemples B1 à B4 ci-dessus où le rendement est de plus de 90%).

## Revendications

1. Dispersion stabilisée de particules minérales au sein d'un milieu dispersant organique, contenant, à titre d'agents de stabilisation de la dispersion, des polymères séquencés amphiphiles P, où lesdits polymères P comprennent:
- un bloc hydrophile A contenant des groupements R^{A} susceptibles de développer des interactions avec la surface des particules ; et
- au moins un bloc hydrophobe B lié au bloc hydrophile A présentant une affinité vis-à-vis du milieu organique dispersant.

2. Dispersion selon la revendication 1, où les particules minérales sont des particules à base d'un oxyde minéral, de préférence SiO₂, CeO₂, TiO₂, ZrO₂, Al₂O₃, Fe₂O₃, ou un mélange de ces oxydes.

3. Dispersion selon la revendication 1 ou selon la revendication 2, où la teneur en particules minérales est comprise entre1 % et 15% en masse par rapport à la masse totale de la dispersion.

4. Dispersion selon l'une quelconque des revendications précédentes, où la taille des objets minéraux qui y sont dispersés, sans prendre en considération la couche d'espèces organiques qui entoure ces objets minéraux, est comprise entre 1 et 70 nm.

5. Dispersion selon l'une quelconque des revendications précédentes, où le milieu dispersant organique est un milieu polaire.

6. Dispersion selon l'une quelconque des revendications précédentes, où les particules minérales ont une surface chargée négativement, et où tout ou partie des groupements R^{A} du bloc A des polymères P sont des groupements de nature cationique, par exemple des groupements ammonium ou amines à l'état quaternisé.

7. Dispersion selon la revendication 6, où les particules minérales sont des particules à base de silice, et où les groupement R^{A} sont des groupements ammonium ou amines à l'état quaternisé.

8. Dispersion selon l'une quelconque des revendications 1 à 5, où les particules minérales ont une surface chargée positivement, et où tout ou partie des groupements R^{A} du bloc A des polymères P sont des groupements de nature anionique.

9. Dispersion selon la revendication 8, où le bloc A des polymères P comporte des groupements carboxylate, sulfate, sulfonate, phosphonate ou phosphate.

10. Dispersion selon la revendication 8 ou la revendication 9, où les particules minérales sont des particules à base d'oxyde de cérium CeO₂.

11. Dispersion selon la revendication **10,** où le bloc A est comporte des groupements -COOH, qui sont de préférence au moins partiellement ionisés à l'état de carboxylates (-COO⁻).

12. Dispersion selon la revendication **11,** où le bloc A est un bloc polyacrylate ou poly(acide acrylique), un bloc poly(styrène sulfonate), un bloc poly(acide vinylphosphonique), ou un bloc poly(acide acrylamido methylpropanesulfonique).

13. Dispersion selon l'une quelconque des revendications précédentes, où les polymères P utilisés à titre d'agents de stabilisation de la dispersion comprennent des polymères diblocs constitués d'une association du bloc hydrophile A et du bloc hydrophobe B, à savoir des polymères de formule schématique A-B.

14. Dispersion selon l'une quelconque des revendications précédentes, dans laquelle les polymères P comprennent des copolymères ayant plusieurs blocs hydrophobes liés de façon covalente au bloc hydrophile A.

15. Dispersion selon la revendication 14, où les copolymères ayant plusieurs blocs hydrophobes liés de façon covalente au bloc hydrophile A comprennent :
- des polymères séquencés de type tribloc, de formule B1-A-B2 où chacun des groupements B1 et B2 représente un bloc hydrophobe de type B tel que défini dans la revendication 1 ; et/ou
- des polymères de type peigne dans lesquels plusieurs blocs hydrophobes de type B tel que défini dans la revendication 1 sont liés, à titre de chaînes latérales, sur le bloc hydrophile A.

16. Dispersion selon l'une quelconque des revendications précédentes, dans laquelle les polymères P comportent statistiquement un nombre moyen de groupement R^{A} supérieur à 1 au sein du bloc A, ce nombre moyen de groupements R^{A} au sein du bloc A étant de préférence au moins égal à 2, par exemple entre 2 et 4.

17. Dispersion selon l'une quelconque des revendications précédentes, dans laquelle le polymère a une masse moléculaire inférieure à 10 000 g/mol, avantageusement entre 1 000 et 3 000 g/mol.

18. Dispersion selon l'une quelconque des revendications précédentes,dans laquelle, dans les polymères P, le rapport massique (bloc hydrophile A / bloc(s) B) est compris entre 0,02 et 0,5.

19. Dispersion selon l'une quelconque des revendications précédentes, dans laquelle la masse moléculaire du bloc A est comprise entre 50 et 5000 g/mol, et où la masse moléculaire de chacun des groupements hydrophobes B est comprise entre 500 et 8000 g/mol.

20. Dispersion selon l'une quelconque des revendications précédentes, dans laquelle le ratio massique (particules/polymère) est supérieur ou égal à 0,2.

21. Dispersion selon l'une quelconque des revendications précédentes, dans laquelle ratio molaire (groupes R^{A} du bloc hydrophile A / constituant minéral des particules) est supérieur ou égal à 0,2.

22. Dispersion selon l'une quelconque des revendications précédentes, dans laquelle le ratio massique (polymères / solvant) est supérieur ou égal à 0,005.

23. Dispersion selon l'une quelconque des revendications précédentes, dans laquelle les polymères P utilisés sont des polymères susceptibles d'être obtenus selon un procédé de polymérisation radicalaire contrôlé comprenant les étapes successives suivantes :
(e1) on forme un premier bloc polymère fonctionnalisé en bout de chaîne en mettant en contact :
- au moins un monomère éthyléniquement insaturé,
- au moins une source de radicaux libres, et
- au moins un xanthate, un dithioester, une thioether-thione ou un dithiocarbamate, de préférence un xanthate; puis
(e2) on forme sur le premier bloc polymère fonctionnalisé en bout de chaîne obtenu dans l'étape (e1) un deuxième bloc, en faisant réagir le polymère obtenu avec un nouveau monomère éthyléniquement insaturé et une source de radicaux libres.

24. Dispersion selon la revendication **23,** où les polymères P utilisés sont obtenus en utilisant des étapes (e1) et (e2) et en mettant en oeuvre des xanthates porteurs de groupement O-ethyle ou O-trifluoroethyle.

25. Dispersion selon la revendication **23** ou **24,** dans laquelle les particules présentes ont une charge de surface négative et où les polymères P sont des polymères séquencés de type poly(acrylate de butyle)-poly(acrylate de 2-dimethylaminoethyle quaternisé) ; poly(acrylate de 2-ethylhexyle)-poly(acrylate de 2-dimethylaminoethyle quaternisé); ou poly(acrylate de 2-ethylhexyle)-poly(p-chloromethyl styrène quaternisé).

26. Dispersion selon la revendication **23** ou **24,** où les particules ont une charge de surface positive et où les polymères P sont des polymères séquencés de type poly(acrylate de butyle)-poly(acide acrylique) ; poly(acrylate de 2-ethylhexyle)-poly(acide acrylique), poly(acrylate de 2-ethylhexyle)-poly(styrène sulfonate); poly(acrylate de butyle)-poly(styrène sulfonate); poly(acrylate d'isooctyle)-poly(acide acrylique) ; ou poly(acrylate de 2-ethyl hexyle)-poly(acide vinyl phosphonique).

27. Dispersion selon la revendication **26,** où les polymères P utilisés sont des polymères séquencés diblocs poly(acide acrylique)-poly(2-ethylhexylacrylate) (PAA-P2EHA).

28. Polymères séquencés diblocs poly(acide acrylique)-poly(2-ethylhexylacrylate) (PAA-P2EHA), utiles à titre d'agents de stabilisation dans une dispersion selon la revendication **27**.

29. Procédé de préparation d'une dispersion selon l'une quelconque des revendications 1 à **27,** où le milieu organique dispersant est de nature hydrophobe, comprenant une étape (A) consistant à mettre en contact une suspension aqueuse des particules avec le milieu organique hydrophobe en présence des polymères séquencés amphiphiles P tels que définis dans la revendication 1, ce par quoi on obtient un transfert des particules de la phase aqueuse vers le milieu hydrophobe sous la forme d'une suspension organique stabilisée par les polymères.

30. Procédé selon la revendication **29,** dans lequel la suspension aqueuse initiale utilisée dans l'étape (A) a une concentration en particules comprise entre 1 et 100 g/L, cette concentration étant de préférence inférieure ou égale à 75 g/L.

31. Procédé selon la revendication **29** ou la revendication **30,** dans lequel les particules dispersées dans la suspension aqueuse initiale utilisée dans l'étape (A) ont un diamètre hydrodynamique moyen compris entre 1 et 200 nm.

32. Composition dispersible en milieu organique pour former une dispersion de particules minérales selon l'une quelconque des revendications 1 à **27,** comprenant des particules minérales et des polymères séquencés amphiphiles P tels que définis dans la revendication 1, ladite composition étant susceptible d'être obtenue par élimination, notamment par évaporation, du milieu organique présent dans une dispersion de particules minérales telle que définie dans l'une quelconque des revendications 1 à **27.**

33. Procédé pour la préparation d'une composition selon la revendication **32,** par évaporation du milieu organique dans une dispersion de particules minérales telle que définie dans l'une des revendications 1 à **27.**

34. Procédé de préparation d'une dispersion de particules minérales selon l'une quelconque des revendications 1 à **27,** dans un milieu organique hydrophobe ou non, ce procédé comprenant une étape (B) où on disperse d'une composition selon la revendication **32** au sein dudit milieu organique.

35. Utilisation d'une dispersion selon l'une quelconque des revendications 1 à **27,** pour la préparation de compositions solvantées, en particulier des peintures ou des compositions cosmétiques.

36. Utilisation d'une dispersion selon l'une quelconque des revendications 1 à **27,** où les particules présentent des propriétés catalytiques, pour effectuer des réactions catalysées en milieu solvant.

## Claims

1. Stabilised dispersion of mineral particles in an organic dispersant medium containing, as stabilisation agents for the dispersion, sequenced amphiphilic polymers P, where said polymers P comprise:
- a hydrophilic block A containing groups R^{A} which are able to develop interactions with the surface of the particles; and
- at least one hydrophobic block B connected to the hydrophilic bloc A and having an affinity with respect to the organic dispersant medium.

2. Dispersion according to claim 1, where the mineral particles are particles based on a mineral oxide, preferably SiO₂, CeO₂, TiO₂, ZrO₂, Al₂O₃, Fe₂O₃, or a mixture of these oxides.

3. Dispersion according to claim 1 or according to claim 2, where the content of mineral particles is between 1% and 15% by mass relative to the total mass of the dispersion.

4. Dispersion according to any of the preceding claims, where the size of the mineral objects which are dispersed there, without taking into account the layer of organic species which surround these mineral objects, is between 1 and 70 nm.

5. Dispersion according to any of the preceding claims, where the organic dispersant medium is a polar medium.

6. Dispersion according to any of the preceding claims, where the mineral particles have a negatively charged surface, and where all or part of the groups R^{A} of the block A of the polymers P are groups of a cationic nature, for example ammonium or amine groups in the quaternised state.

7. Dispersion according to claim 6, where the mineral particles are particles based on silica, and where the groups R^{A} are ammonium or amine groups in the quaternised state.

8. Dispersion according to any of the claims 1 to 5, where the mineral particles have a positively charged surface and where all or part of the groups R^{A} of the block A of the polymers P are groups of an anionic nature.

9. Dispersion according to claim 8, where the block A of the polymers P comprises carboxylate, sulphate, sulphonate, phosphonate or phosphate groups.

10. Dispersion according to claim 8 or claim 9, where the mineral particles are particles based on cerium oxide CeO₂.

11. Dispersion according to claim 10, where the bloc A is made up of -COOH groups, which are preferably at least partially ionised to the state of carboxylates (-COO-).

12. Dispersion according to claim 11, where the block A is a polyacrylate or poly(acrylic acid) block, a poly(styrene sulphonate) block, a poly(vinylphosphonic acid) block or a poly(acrylamidomethylpropanesulphonic acid) block.

13. Dispersion according to any of the preceding claims, where the polymers P which are used as stabilisation agents for the dispersion comprise diblock polymers comprising an association of the hydrophilic block A and of the hydrophobic block B, i.e. polymers of the schematic formula A-B.

14. Dispersion according to any of the preceding claims, in which the polymers P comprise copolymers with several hydrophobic blocks connected to the hydrophilic block A in a covalent manner.

15. Dispersion according to claim 14, where the copolymers having several hydrophobic groups connected to the hydrophilic block A in a covalent manner comprise:
- sequenced polymers of the triblock type, of formula B1-A-B2 where each of the groups B1 and B2 represents a hydrophobic block of type B as defined in claim 1; and/or
- polymers of the comb type in which several hydrophobic blocks of type B as defined in claim 1 are connected, as lateral chains, on the hydrophilic block A.

16. Dispersion according to any of the preceding claims, in which the polymers P comprise statistically an average group number R^{A} greater than 1 in block A, this average number of groups R^{A} in block A being preferably at least equal to 2, for example between 2 and 4.

17. Dispersion according to any of the preceding claims, in which the polymer has a molecular mass less than 10,000 g/mol, advantageously between 1,000 and 3,000 g/mol.

18. Dispersion according to any of the preceding claims, in which, in the polymers P, the mass ratio (hydrophilic block A/block(s) B) is between 0.02 and 0.5.

19. Dispersion according to any of the preceding claims, in which the molecular mass of the block A is between 50 and 5,000 g/mol, and where the molecular mass of each of the hydrophobic groups B is between 500 and 8,000 g/mol.

20. Dispersion according to any of the preceding claims, in which the mass ratio (particles/polymer) is greater than or equal to 0.2.

21. Dispersion according to any of the preceding claims, in which molar ratio (groups R^{A} of the hydrophilic block A/mineral constituent of the particles) is greater than or equal to 0.2.

22. Dispersion according to any of the preceding claims, in which the mass ratio (polymers/ solvent) is greater than or equal to 0.005.

23. Dispersion according to any of the preceding claims, in which the polymers P which are used are polymers which can be obtained according to a controlled radical polymerisation process comprising the following successive steps:
(e1) a first polymer block is formed, functionalised at the chain end, by bringing into contact:
- at least one ethylenically unsaturated monomer,
- at least one source of free radicals, and
- at least one xanthate, one dithioester, one thioether-thione or one dithiocarbamate, preferably a xanthate; then
(e2) a second block is formed on the first polymer block, functionalised at the chain end and obtained in step (e1), by causing the obtained polymer to react with a new ethylenically unsaturated monomer and a source of free radicals.

24. Dispersion according to claim 23, where the polymers P which are used are obtained by using steps (e1) and (e2) and by using xanthates which are carriers of an O-ethyl or O-trifluoroethyl group.

25. Dispersion according to claim 23 or 24, in which the particles which are present have a negative surface charge and where the polymers P are sequenced polymers of the type poly(butyl acrylate)-poly(quaternised 2-dimethylaminoethyl acrylate); poly(2-ethylhexyl acrylate)-poly(quaternised 2-dimethylaminoethyl acrylate); or poly(2-ethylhexyl acrylate)-poly(quaternised p-chloromethyl styrene).

26. Dispersion according to claim 23 or 24, where the particles have a positive surface charge and where the polymers P are sequenced polymers of the type poly(butyl acrylate)-poly(acrylic acid); poly(2-ethylhexyl acrylate)-poly(acrylic acid), poly(2-ethylhexyl acrylate)-poly(styrene sulphonate); poly(butyl acrylate)-poly(styrene sulphonate); poly(isooctyl acrylate)-poly(acrylic acid); or poly(2-ethylhexyl acrylate)-poly(vinyl phosphonic acid).

27. Dispersion according to claim 26, where the polymers P which are used are poly(acrylic acid)-poly(2-ethylhexyl acrylate) (PAA-P2EHA) dibloc sequenced polymers.

28. Poly(acrylic acid)-poly(2-ethylhexyl acrylate) (PAA-P2EHA) dibloc sequenced polymers which are useful as stabilisation agents in a dispersion according to claim 27.

29. Method for preparing a dispersion according to any of the claims 1 to 27, where the organic dispersant medium is of a hydrophobic nature, comprising a step (A) which comprises bringing an aqueous suspension of particles into contact with the hydrophobic organic medium in the presence of amphiphilic sequenced polymers P as defined in claim 1, by means of which a transfer of the particles of the aqueous phase to the hydrophobic medium is obtained in the form of an organic suspension which is stabilised by the polymers.

30. Method according to claim 29, in which the initial aqueous suspension which is used in step (A) has a concentration of particles between 1 and 100 g/l, this concentration being preferably less than or equal to 75 g/l.

31. Method according to claim 29 or claim 30, in which the particles which are dispersed in the initial aqueous suspension which is used in step (A) have a mean hydrodynamic diameter between 1 and 200 nm.

32. Composition which is dispersible in organic medium in order to form a dispersion of mineral particles according to any of the claims 1 to 27, comprising mineral particles and amphiphilic sequenced polymers P as defined in claim 1, said composition being able to be obtained by elimination, in particular by evaporation, of the organic medium which is present in a dispersion of mineral particles as defined in any of the claims 1 to 27.

33. Method for preparing a composition according to claim 32, by evaporation of the organic medium in a dispersion of mineral particles as defined in one of the claims 1 to 27.

34. Method for preparing a dispersion of mineral particles according to any of the claims 1 to 27, in a hydrophobic or non-hydrophobic organic medium, this method comprising a step (B) where a composition according to claim 32 is dispersed in said organic medium.

35. Use of a dispersion according to any of the claims 1 to 27, for the preparation of solvent compositions, in particular paints or cosmetic compositions.

36. Use of a dispersion according to any of the claims 1 to 27, where the particles have catalytic properties, in order to effect reactions which are catalysed in a solvent medium.

## Patentansprüche

1. Stabilisierte Dispersion von mineralischen Teilchen in einem dispergierenden organischen Medium, die als Mittel zur Stabilisierung der Dispersion amphiphile sequenzierte Polymere P enthält und bei der die Polymere P umfassen:
- einen hydrophilen Block A, der Gruppen R^{A} enthält, die Wechselwirkungen mit der Oberfläche der Teilchen entwickeln können; und
- mindestens einen an den hydrophilen Block A gebundenen hydrophoben Block B, der eine Affinität zu dem dispergierenden organischen Medium besitzt.

2. Dispersion nach Anspruch 1, bei der die mineralischen Teilchen Teilchen auf Basis eines mineralischen Oxids, insbesondere SiO₂, CeO₂, TiO₂, ZrO₂, Al₂O₃, Fe₂O₃, oder eine Mischung dieser Oxide sind.

3. Dispersion nach Anspruch 1 oder nach Anspruch 2, bei der der Gehalt an mineralischen Teilchen zwischen 1 und 15 Masseprozent, bezogen auf die Gesamtmasse der Dispersion, beträgt.

4. Dispersion nach einem der vorhergehenden Ansprüche, bei der die Größe der in ihr dispergierten mineralischen Objekte, ohne Berücksichtigung der Schicht von organischen Spezies, die diese mineralischen Objekte umgibt, zwischen 1 und 70 nm beträgt.

5. Dispersion nach einem der vorhergehenden Ansprüche, bei der das dispergierende organische Medium ein polares Medium ist.

6. Dispersion nach einem der vorhergehenden Ansprüche, bei der die mineralischen Teilchen eine negativ geladene Oberfläche besitzen und bei der alle Gruppen R^{A} des Blocks A der Polymere P oder ein Teil davon Gruppen von kationischer Natur sind, beispielsweise Ammonium- oder Amingruppen in quaternisiertem Zustand.

7. Dispersion nach Anspruch 6, bei der die mineralischen Teilchen Teilchen auf Siliciumoxidbasis sind und bei der die Gruppen R^{A} Ammonium- oder Amingruppen in quaternisiertem Zustand sind.

8. Dispersion nach einem der Ansprüche 1 bis 5, bei der die mineralischen Teilchen eine positiv geladene Oberfläche besitzen und bei der alle Gruppen R^{A} des Blocks A der Polymere P oder ein Teil davon Gruppen von anionischer Natur sind.

9. Dispersion nach Anspruch 8, bei der der Block A der Polymere P Carboxylat-, Sulfat-, Sulfonat-, Phosphonat- oder Phosphatgruppen umfasst.

10. Dispersion nach Anspruch 8 oder Anspruch 9, bei der die mineralischen Teilchen Teilchen auf Basis von Ceroxid CeO₂ sind.

11. Dispersion nach Anspruch 10, bei der der Block A -COOH-Gruppen umfasst, die vorzugsweise mindestens teilweise ionisiert im Zustand von Carboxylaten (-COO⁻) sind.

12. Dispersion nach Anspruch 11, bei der der Block A ein Polyacrylat- oder Poly(acrylsäure)-Block, ein Poly(styrolsulfonat)-Block, ein Poly(vinylphosphonsäure)-Block oder ein Poly(acrylamidomethylpropansulfonsäure)-Block ist.

13. Dispersion nach einem der vorhergehenden Ansprüche, bei der die als Mittel zur Stabilisierung der Dispersion verwendeten Polymere P Diblockpolymere umfassen, die aus einer Kombination des hydrophilen Blocks A und des hydrophoben Blocks B bestehen, und zwar Polymere der schematischen Formel A-B.

14. Dispersion nach einem der vorhergehenden Ansprüche, bei der die Polymere P Copolymere umfassen, die mehrere hydrophobe Blöcke aufweisen, die an den hydrophilen Block A kovalent gebunden sind.

15. Dispersion nach Anspruch 14, bei der die Copolymere, die mehrere hydrophobe Blöcke aufweisen, die an den hydrophilen Block A kovalent gebunden sind, umfassen:
- sequenzierte Triblockpolymere der Formel B1-A-B2, worin jede der Gruppen B1 und B2 einen hydrophoben Block vom Typ B, wie er in Anspruch 1 definiert ist, darstellt; und/oder
- Kammpolymere, bei denen mehrere hydrophobe Blöcke vom Typ B, wie er in Anspruch 1 definiert ist, als Seitenketten an den hydrophilen Block A gebunden sind.

16. Dispersion nach einem der vorhergehenden Ansprüche, bei der die Polymere P statistisch eine mittlere Anzahl von Gruppen R^{A} größer als 1 in dem Block A umfassen, wobei diese mittlere Anzahl von Gruppen R^{A} in dem Block A vorzugsweise mindestens gleich 2, beispielsweise zwischen 2 und 4, ist.

17. Dispersion nach einem der vorhergehenden Ansprüche, bei der das Polymer eine Molmasse unter 10 000 g/mol, vorteilhafterweise zwischen 1 000 und 3 000 g/mol, besitzt.

18. Dispersion nach einem der vorhergehenden Ansprüche, bei der in den Polymeren P das Masseverhältnis (hydrophiler Block A / Block bzw. Blöcke B) zwischen 0,02 und 0,5 beträgt.

19. Dispersion nach einem der vorhergehenden Ansprüche, bei der die Molmasse des Blocks A zwischen 50 und 5000 g/mol beträgt und die Molmasse jeder der hydrophoben Gruppen B zwischen 500 und 8000 g/mol beträgt.

20. Dispersion nach einem der vorhergehenden Ansprüche, bei der das Masseverhältnis (Teilchen/Polymer) größer als oder gleich 0,2 ist.

21. Dispersion nach einem der vorhergehenden Ansprüche, bei der das Molverhältnis (Gruppen R^{A} des hydrophilen Blocks A / minderalischer Bestandteil der Teilchen) größer als oder gleich 0,2 ist.

22. Dispersion nach einem der vorhergehenden Ansprüche, bei der das Molverhältnis (Polymere/Lösungsmittel) größer als oder gleich 0,005 ist.

23. Dispersion nach einem der vorhergehenden Ansprüche, bei der die verwendeten Polymere P Polymere sind, die in einem Verfahren zur gesteuerten radikalischen Polymerisation hergestellt werden können, das die folgenden aufeinanderfolgenden Schritte umfasst:
(e1) man bildet einen ersten am Kettenende funktionalisierten Polymerblock, indem man in Kontakt bringt:
- mindestens ein ethylenisch ungesättigtes Monomer,
- mindestens eine Quelle von freien Radikalen und
- mindestens ein Xanthat, einen Dithioester, ein Thioetherthion oder ein Dithiocarbamat, vorzugsweise ein Xanthat; und dann
(e2) bildet man an dem im Schritt (e1) erhaltenen ersten am Kettenende funktionalisierten Polymerblock einen zweiten Block, indem man das erhaltene Polymer mit einem neuen ethylenisch ungesättigten Monomer und einer Quelle von freien Radikalen reagieren lässt.

24. Dispersion nach Anspruch 23, bei der die verwendeten Polymere P hergestellt werden, indem Schritte (e1) und (e2) verwendet werden und indem O-Ethyl- oder O-Trifluorethylgruppen tragende Xanthate eingesetzt werden.

25. Dispersion nach Anspruch 23 oder 24, bei der die vorhandenen Teilchen eine negative Oberflächenladung aufweisen und bei der die Polymere P sequenzierte Polymere vom Typ Poly(butylacrylat)-poly(quaternisiertes 2-dimethylaminoethylacrylat); Poly(2-ethylhexylacrylat)-poly(quaternisertes 2-dimethylaminoethylacrylat); oder Poly(2-ethylhexylacrylat)-poly( quaternisiertes p-chlormethylstyrol) sind.

26. Dispersion nach Anspruch 23 oder 24, bei der die Teilchen eine positive Oberflächenladung aufweisen und bei der die Polymere P sequenzierte Polymere vom Typ Poly(butylacrylat)-poly(acrylsäure); Poly(2-ethylhexylacrylat)-poly(acrylsäure), Poly(2-ethylhexylacrylat)-poly(styrolsulfonat); Poly(butylacrylat)-poly(styrolsulfonat); Poly(isooctylacrylat)-poly(acrylsäure); oder Poly(2-ethylhexylacrylat)-poly(vinylphosphonsäure) sind.

27. Dispersion nach Anspruch 26, bei der die verwendeten Polymere P sequenzierte Diblockpolymere Poly(acrylsäure)-poly(2-ethylhexylacrylat) (PAA-P2EHA) sind.

28. Sequenzierte Diblockpolymere Poly(acrylsäure)-poly(2-ethylhexylacrylat) (PAA-P2EHA), die als Stabilisierungsmittel in einer Dispersion nach Anspruch 27 verwendbar sind.

29. Verfahren zur Herstelllung einer Dispersion nach einem der Ansprüche 1 bis 27, bei dem das dispergierende organische Medium von hydrophober Natur ist, umfassend einen Schritt (A), der darin besteht, dass eine wässrige Suspension der Teilchen mit dem hydrophoben organischen Medium in Gegenwart der amphiphilen sequenzierten Polymere P, wie sie in Anspruch 1 definiert sind, in Kontakt gebracht wird, wodurch man einen Transfer der Teilchen von der wässrigen Phase zum hydrophoben Medium in Form einer durch die Polymere stabilisierten organischen Suspension erhält.

30. Verfahren nach Anspruch 29, bei dem die im Schritt (A) verwendete ursprüngliche wässrige Lösung eine Konzentration an Teilchen zwischen 1 und 100 g/l aufweist, wobei diese Konzentration vorzugsweise kleiner als oder gleich 75 g/l ist.

31. Verfahren nach Anspruch 29 oder 30, bei dem die Teilchen, die in der im Schritt (A) verwendeten ursprünglichen wässrigen Suspension dispergiert sind, einen mittleren hydrodynamischen Durchmesser zwischen 1 und 200 nm aufweisen.

32. Zusammensetzung, die in einem organischen Medium dispergierbar ist, um eine Dispersion von mineralischen Teilchen nach einem der Ansprüche 1 bis 27 zu bilden, umfassend mineralische Teilchen und amphiphile sequenzierte Polymere P, wie sie in Anspruch 1 definiert sind, wobei diese Zusammensetzung durch Entfernung, insbesondere durch Abdampfen, des organischen Mediums erhalten werden kann, das in einer Dispersion von mineralischen Teilchen, wie sie in einem der Ansprüche 1 bis 27 definiert ist, vorhanden ist.

33. Verfahren zur Herstellung einer Zusammensetzung nach Anspruch 32 durch Abdampfen des organischen Mediums in einer Dispersion von mineralischen Teilchen, wie sie in einem der Ansprüche 1 bis 27 definiert ist.

34. Verfahren zur Herstellung einer Dispersion von mineralischen Teilchen nach einem der Ansprüche 1 bis 27 in einem hydrophoben oder nicht hydrophoben organischen Medium, wobei dieses Verfahren einen Schritt (B) umfasst, in dem man eine Zusammensetzung nach Anspruch 32 in dem organischen Medium dispergiert.

35. Verwendung einer Dispersion nach einem der Ansprüche 1 bis 27 für die Herstellung von lösungsmittelhaltigen Zusammensetzungen, insbesondere Anstrichfarben oder kosmetischen Zusammensetzungen.

36. Verwendung einer Dispersion nach einem der Ansprüche 1 bis 27, bei der die Teilchen katalytische Eigenschaften zur Durchführung von katalysierten Reaktionen in Lösungsmittelmedium aufweisen.
